(19) Europäisches Patentamt
European Patent Office
Office européen des brevets

(11) EP 3 533 444 A1

(12) # EUROPEAN PATENT APPLICATION
published in accordance with Art. 153(4) EPC

(43) Date of publication:
**04.09.2019 Bulletin 2019/36**

(21) Application number: **17864783.0**

(22) Date of filing: **27.10.2017**

(51) Int Cl.:
***A61K 31/135*** *(2006.01)* ***A61K 31/357*** *(2006.01)*
***A61K 31/661*** *(2006.01)* ***A61P 25/22*** *(2006.01)*
***A61P 25/24*** *(2006.01)* ***C07C 211/29*** *(2006.01)*
***C07D 317/24*** *(2006.01)*

(86) International application number:
**PCT/JP2017/038826**

(87) International publication number:
**WO 2018/079693 (03.05.2018 Gazette 2018/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.10.2016 JP 2016210749**

(71) Applicant: **National University Corporation Chiba University**
**Chiba-shi, Chiba 267-8522 (JP)**

(72) Inventor: **HASHIMOTO, Kenji**
**Chiba-shi**
**Chiba 260-8670 (JP)**

(74) Representative: **Cooley (UK) LLP**
**Dashwood**
**69 Old Broad Street**
**London EC2M 1QS (GB)**

(54) **PHARMACEUTICAL APPLICATIONS FOR (S)-NORKETAMINE AND SALTS THEREOF**

(57) To provide: an agent for the prevention and/or treatment of a depressive symptom, the drug consisting of (S)-norketamine or a pharmacologically acceptable salt thereof; a pharmaceutical composition for the prevention and/or treatment of a depressive symptom, the pharmaceutical composition containing (S)-norketamine or a pharmacologically acceptable salt thereof in an amount effective for alleviation of the depressive symptom, and being substantially free of (R)-norketamine; a method for the prevention and/or treatment of a depressive symptom, the method including administration of the drug or the composition; and use of (S)-norketamine for production of a drug for the prevention and/or treatment of a depressive symptom or a composition for the prevention and/or treatment of a depressive symptom.

EP 3 533 444 A1

## Description

### Technical Field

The present invention relates to a pharmaceutical for the prevention and/or treatment of psychiatric diseases, preferably diseases exhibiting depressive symptoms. More particularly, the present invention relates to an antidepressant consisting essentially of (S)-norketamine (i.e., an optical isomer of norketamine (N-desmethylketamine)), a prodrug of (S)-norketamine, or a pharmacologically acceptable salt of any of these, and to a pharmaceutical composition for the prevention and/or treatment of diseases exhibiting depressive symptoms, the pharmaceutical composition containing (S)-norketamine, a prodrug of (S)-norketamine, or a pharmacologically acceptable salt of any of these.

The present application claims priority to Japanese Patent Application No. 2016-210749, which is incorporated herein by reference.

### Background Art

In association with changes in social life style or aging of society, various diseases such as psychiatric diseases and neurological diseases tend to increase as a whole. For example, incidences of depression and schizophrenia, which are typical psychiatric diseases, are high, and have become a serious problem from the viewpoint of medical economics. Furthermore, Obsessive-compulsive disorder is one anxiety disorder involving obsessions and compulsions. The treatment of a psychiatric disease (e.g., depression, schizophrenia, or anxiety disorder) requires drug therapy, and involves administration of an antidepressant (e.g., a tricyclic antidepressant, a selective serotonin reuptake inhibitor, or a serotonin and norepinephrine reuptake inhibitor) or an antipsychotic (e.g., a phenothiazine compound, a butyrophenone compound, a benzamide compound, an iminodibenzyl compound, a thiepin compound, an indole compound, or a serotonin/dopamine receptor antagonist). However, although these drugs, which are actually used in clinical fields, are effective for some patients and some symptoms, patients for whom the drugs are ineffective (so-called treatment-resistant patients) are also known to exist. Thus, a strong demand has arisen for development of a new therapeutic drug. It is hard to say that the existing drugs exhibit sufficient therapeutic effects on these psychiatric diseases. In fact, virtually no preventive or therapeutic method is available for the diseases.

One of the major problems in treatment of depression is that there are limits to the effects of antidepressants and to the effects of adjuvant therapy of depression. Several weeks or more are required for current antidepressants to express their drug efficacy, and there exist treatment-resistant patients for whom the antidepressants are ineffective. Thus, it is said that only a mere 50% of patients with depression reach remission. When the dose of such an antidepressant is increased for remission of a patient with depression, the patient suffers from various side effects. Furthermore, depression is one of the causes of suicide.

In recent research, growing evidence suggests that abnormality in glutamatergic transmission, in particular, glutamatergic neurotransmission via an N-methyl-D-aspartate (hereinafter abbreviated as "NMDA") receptor, is associated with pathophysiology of mood disorders (e.g., depression and bipolar disorder), and the NMDA receptor also plays important roles in neurobiology and treatment of major depressive disorder (hereinafter abbreviated as "MDD") (Non-Patent Document 1).

Ketamine, which is an NMDA receptor antagonist, has been reported to exhibit a rapid and potent antidepressant effect on treatment-resistant MDD patients and depressive symptoms of treatment-resistant bipolar disorder (Non-Patent Documents 2 to 4). Also, ketamine has been reported to be effective for treatment-resistant obsessive-compulsive disorder and treatment-resistant posttraumatic stress disorder (hereinafter abbreviated as "PTSD") (Non-Patent Documents 5 to 7).

Currently, ketamine is one of the drugs that have received attention for the treatment of treatment-resistant MDD patients, depressive symptoms of treatment-resistant bipolar disorder, treatment-resistant obsessive-compulsive disorder, treatment-resistant PTSD, and autism spectrum disorder (Non-Patent Documents 4 to 9).

Ketamine, which is a compound developed as an anesthetic in 1962, started to be applied clinically in 1965. However, ketamine is designated as a scheduled drug because of its problems of psychotic symptoms (e.g., hallucination and delusion) and drug dependence. Thus, ketamine has been used as an anesthetic and for the treatment of chronic pain in clinical fields.

It has been reported that a clinical antidepressant effect of ketamine lasts for a short period of one to two days starting from several hours after its single administration. Meanwhile, it has been reported that the effect may last two weeks or longer (Non-Patent Documents 2, 3, and 8). It has also been reported that ketamine has a psychotomimetic effect as a side effect, and the antidepressant effect of ketamine is not represented until disappearance of the side effect (Non-Patent Documents 2 and 3).

Ketamine is in a form of a racemic mixture containing equal amounts of (R)-ketamine and (S)-ketamine, which are optical isomers. The present inventors have disclosed that (R)-ketamine or a pharmaceutically acceptable salt thereof

has a rapid and long-lasting antidepressant effect and is less likely to exhibit side effects observed in (S)-ketamine, and thus (R)-ketamine or a pharmaceutically acceptable salt thereof is effective for the prevention and/or treatment of psychiatric diseases exhibiting depressive symptoms (Patent Documents 1 and 2 and Non-Patent Documents 10 and 11).

Norketamine, which is a major metabolite of ketamine, has been reported to have lower affinity for an NMDA receptor than ketamine by a factor of about 6.8-fold (Non-Patent Document 12). Similarly to ketamine, norketamine is known to have optical isomers, which are called (R)-norketamine and (S)-norketamine.

Additionally, norketamine has been reported to exhibit an antidepressant effect, although the effect is lower than that of ketamine (Non-Patent Document 13).

Prior Art Documents

Patent Documents

Patent Document 1: Japanese Unexamined Patent Application Publication No. 2015-078181
Patent Document 2: International Publication WO No. 2015/037248 pamphlet
Patent Document 3: US Patent No. 6040479 specification


Non-Patent Documents

Non-Patent Document 1: Hashimoto K (2009) Emerging role of glutamate in the pathophysiology of major depressive disorder. Brain Res. Rev. 61: 105-23.

Non-Patent Document 2: Berman RM, Cappiello A, An and A, Oren DA, Heninger GR, Charney DS, Krystal JH (2000) Antidepressant effects of ketamine in depressed patients. Biol. Psychiatry 47: 351-4.

Non-Patent Document 3: Zarate CA, Jr, Singh JB, Carlson PJ, Brutsche NE, Ameli R, Luckenbaugh DA, Charney DS, Manji HK (2006) A randomized trial of an N-methyl-D-aspartate antagonist in treatment-resistant major depression. Arch. Gen. Psychiatry 63: 856-64.

Non-Patent Document 4: Diazgranados N, Ibrahim L, Brutsche NE, Newberg A, Kronstein P, Khalife S, Kammerer WA, Quezado Z, Luckenbaugh DA, Salvadore G, Machado-Vieira R, Manji HK, Zarate CA Jr. (2010) A randomized add-on trial of an N-methyl-D-aspartate antagonist in treatment-resistant bipolar depression. Arch. Gen. Psychiatry 67: 793-802.

Non-Patent Document 5: Bloch MH, Wasylink S, Landeros-Weisenberger A, Panza KE, Billingslea E, Leckman JF, Krystal JH, Bhagwagar Z, Sanacora G, Pittenger C (2012) Effects of ketamine in treatment-refractory obsessive-compulsive disorder. Biol. Psychiatry 72 (11): 964-970.

Non-Patent Document 6: Rodriguez CI, Kegeles LS, Levinson A, Feng T, Marcus SM, Vermes D, Flood P, Simpson HB (2013) Randomized Controlled Crossover Trial of Ketamine in Obsessive-Compulsive Disorder: Proof-of-Concept. Neuropsychopharmacology 38 (12): 2475-2483.

Non-Patent Document 7: Feder A, Parides MK, Murrough JW, Perez AM, Morgan JE, Saxena S, Kirkwood K, Aan Het Rot M, Lapidus KA, Wan LB, Iosifescu D, Charney DS (2014) Efficacy of intravenous ketamine for treatment of chronic posttraumatic stress disorder: a randomized clinical trial. JAMA Psychiatry 71: 681-688.

Non-Patent Document 8: Krystal JH, Sanacora G, Duman RS (2013) Rapid-acting glutamatergic antidepressants: the path to ketamine and beyond. Biol. Psychiatry 73: 1133-41.

Non-Patent Document 9: Wink LK, O'Melia AM, Shaffer RC, Pedapati E, Friedmann K, Schaefer T, Erickson CA (2014) Intranasal ketamine treatment in an adult with autism spectrum disorder. J. Clin. Psychiatry 75 (8): 835-836.

Non-Patent Document 10: Zhang JC, Li SX, Hashimoto K (2014) R(-)-Ketamine shows greater potency and longer lasting antidepressant effects than S(+)-ketamine. Pharmacol. Biochem. Behav. 116: 137-141.

Non-Patent Document 11: Yang C, Shirayama Y, Zhang JC, Ren Q, Yao W, Ma M, Dong C, Hashimoto K (2015) R-Ketamine: a rapid-onset and sustained antidepressant without psychotomimetic side effects. Transl. Psychiatry 5: e632.

Non-Patent Document 12: Ebert B, Mikkelsen S, Thorkildsen C, Bordbjerg FM (1997) Norketamine, the main metabolite of ketamine, is a non-competitive NMDA receptor antagonist in the rat cortex and spinal cord. Eur. J. Pharmacology 333: 99-104.

Non-Patent Document 13: Sarat K, Siwek A, Staroxicz G, Librowski T, Nowak G, Drabik U, Gajdosz R, Popik P (2015) Antidepressant-like effects of ketamine, norketamine and dehydronorketamine in forced swim test: Role of activity at NMDA receptor. Neuropharmacology 99: 301-307.

Non-Patent Document 14: Ma M, Ren Q, Zhang JC, Hashimoto K (2014) Effects of brilliant blue G on serum levels of tumor necrosis factor-alpha and depression-like behaviors in mice after administration of lipopolysaccharide. Clin. Psychopharmacol. Neurosci. 12: 31-36.

Non-Patent Document 15: Zhang JC, Wu J, Fujita Y, Yao W, Ren Q, Yang C, Li SX, Shirayama Y, Hashimoto K (2015) Antidepressant effects of TrkB ligands on depression-like behavior and dendritic changes in the hippocampus and nucleus accumbens after inflammation. Int. J. Neuropsychopharmacol. 18: pyu077.

Non-Patent Document 16: Yao W, Zhang JC, Dong C, Zhuang C, Hirota S, Inanaga K, Hashimoto K (2015) Effects of amycenone on serum levels of tumor necrosis factor-alpha and depression-like behaviors in mice after administration of lipopolysaccharide. Pharmacol. Biochem. Behav. 136: 7-12.

Non-Patent Document 17: Biermann M, Zheng G, Hojahmat M, Moskalev NV, Crooks PA (2015) Asymmetric synthesis of (S)- and (R)-norketamine via Sharpless asymmetric dihydroxylation/Ritter amination sequence. Tetrahedron Letters 56: 2608-2610.

Non-Patent Document 18: Rautio J, Kumpulainen H, Heimbach T, Oliyai R, Oh D, Jarvinen T, Savolainen J (2008) Prodrugs: design and clinical applications. Nat. Rev. Drug Discov. 7 (3): 255-270.

Non-Patent Document 19: Simplicio AL, Clancy JM, Gilmer (2008) Prodrugs for amines. Molecules 13: 519-547.

## Summary of the Invention

### Problems to be Solved by the Invention

Ketamine, which is an NMDA receptor antagonist, has been reported to exhibit a rapid antidepressant effect on treatment-resistant depressive patients. However, since ketamine has problems of side effects (e.g., psychotic symptoms such as hallucination and delusion) and dependence, and is designated as a scheduled drug, difficulty is encountered in practically using ketamine in clinical fields.

An object of the present invention is to provide a novel compound having a long-lasting therapeutic effect on diseases exhibiting depressive symptoms.

### Means for Solving the Problems

The present inventor has conducted extensive studies for solving the aforementioned problems, and has focused on norketamine, which is a major metabolite of ketamine (NMDA receptor antagonist) and had not been used in studies on the antidepressant effect of ketamine.

Depression is considered to be associated with glutamatergic neurotransmission via an NMDA receptor, and ketamine has been reported to exhibit a rapid antidepressant effect on treatment-resistant depressive patients. Furthermore, it is generally understood that the analgesic and psychotomimetic effects of ketamine are both mainly mediated by blocking of an NMDA receptor.

Meanwhile, nobody has paid attention to the pharmacological action of norketamine (i.e., a major metabolite of ketamine) as an antidepressant, since norketamine has lower affinity for an NMDA receptor than ketamine. Furthermore, the psychotomimetic effect of norketamine is expected to be lower than that of ketamine, since norketamine has lower affinity for an NMDA receptor.

The present inventor has found that norketamine exhibits a more potent antidepressant effect than ketamine in studies using model mice exhibiting depression-like symptoms. Meanwhile, side effects (e.g., hyperlocomotion effect and prepulse inhibition deficit) recognized through administration of ketamine are reduced in the case of administration of norketamine. Since norketamine has lower affinity for an NMDA receptor than ketamine, norketamine is considered to have a less psychotomimetic effect (i.e., a side effect) and furthermore, to barely form drug dependence.

In addition, in a social defeat stress model with depression, (S)-norketamine exhibited an antidepressant effect, but (R)-norketamine did not exhibit an antidepressant effect. Furthermore, (S)-norketamine exhibited a more potent antidepressant effect than (R)-norketamine. Additionally, (S)-norketamine did not induce a hyperlocomotion effect, prepulse inhibition deficit, or a rewarding effect. Furthermore, Norketamine and (S)-norketamine, which are not designated as scheduled drugs, are easy to use in clinical fields, as compared with ketamine, which is designated as a scheduled drug. The present invention has been accomplished on the basis of these findings.

That is, the present invention consists of the following.

1. An agent for prevention and/or treatment of a depressive symptom, the agent consisting essentially of (S)-norketamine, a prodrug of (S)-norketamine, or a pharmacologically acceptable salt of any of these.
2. The agent for prevention and/or treatment of a depressive symptom according to 1 above, wherein the depressive symptom is a depressive symptom in depression, a depressive symptom in obsessive-compulsive disorder, a depressive symptom in posttraumatic stress disorder (PTSD), or a depressive symptom in autism spectrum disorder.

3. The agent for prevention and/or treatment of a depressive symptom according to 1 or 2 above, wherein the pharmacologically acceptable salt of (S)-norketamine is (S)-norketamine hydrochloride.
4. The agent for prevention and/or treatment of a depressive symptom according to 1 or 2 above, wherein the prodrug of (S)-norketamine is a compound represented by the following formula (VI), (VII), (XIII), or (XIV):

[F1]

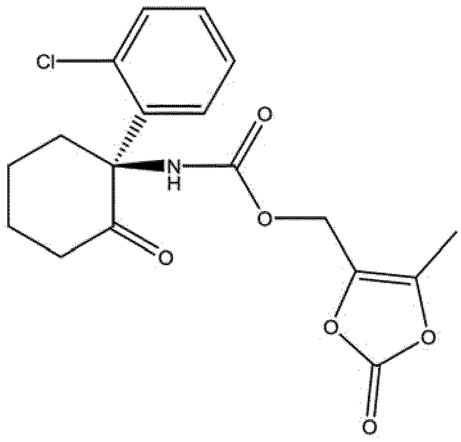

(XIII)

[F2]

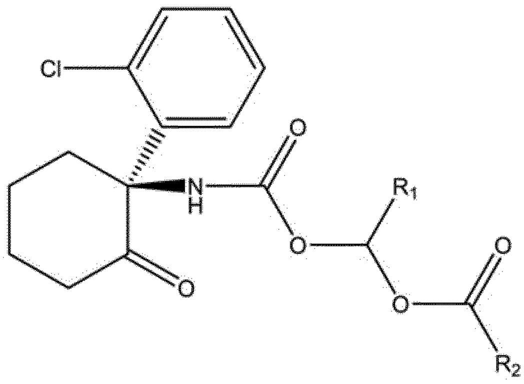

(VI)

[F3]

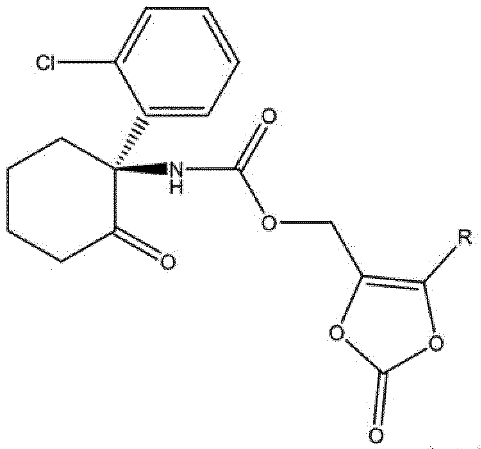

(VII)

[F4]

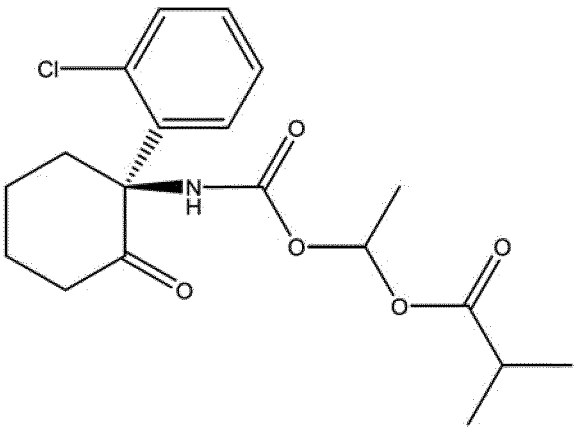

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group), or a pharmacologically acceptable salt or hydrochloride of the compound.

5. A pharmaceutical composition for prevention and/or treatment of a depressive symptom, the pharmaceutical composition comprising (S)-norketamine, a prodrug of (S)-norketamine, or a pharmacologically acceptable salt of any of these in an amount effective for alleviation of the depressive symptom, and being substantially free of (R)-norketamine or a pharmacologically acceptable salt thereof.

6. The pharmaceutical composition for prevention and/or treatment of a depressive symptom according to 5 above, wherein the depressive symptom is a depressive symptom in depression, a depressive symptom in obsessive-compulsive disorder, a depressive symptom in PTSD, or a depressive symptom in autism spectrum disorder.

7. The pharmaceutical composition for prevention and/or treatment of a depressive symptom according to 5 or 6 above, wherein the pharmacologically acceptable salt of (S)-norketamine is (S)-norketamine hydrochloride.

8. The pharmaceutical composition for prevention and/or treatment of a depressive symptom according to 5 or 6 above, wherein the prodrug of (S)-norketamine is a compound represented by the following formula (VI), (VII), (XIII), or (XIV):

[F5]

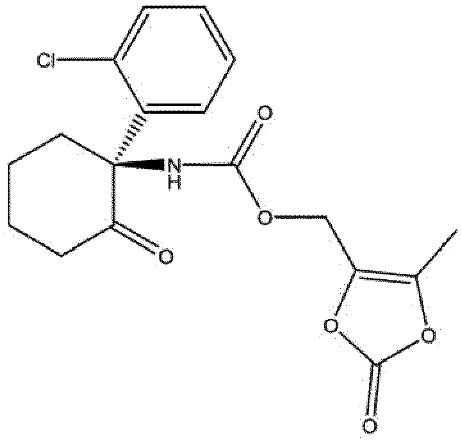

(XIII)

[F6]

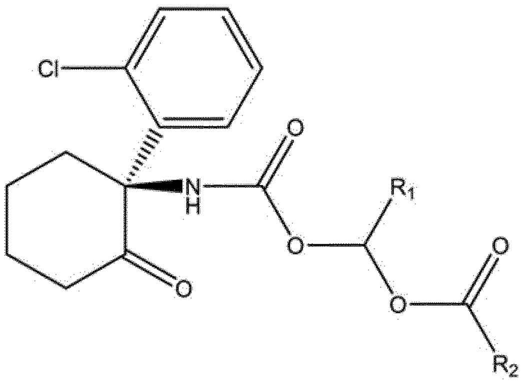

(VI)

[F7]

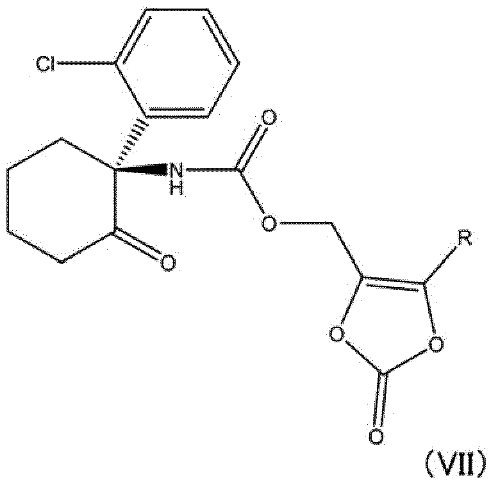

(VII)

[F8]

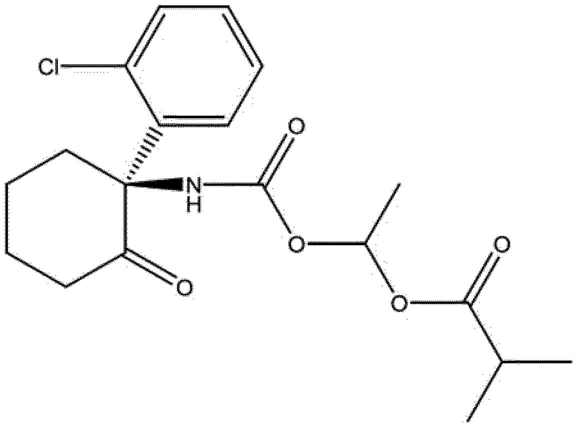

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group), or a pharmacologically acceptable salt or hydrochloride of the compound.

9. A method for prevention and/or treatment of a depressive symptom, the method comprising administering, to a patient in need of prevention and/or treatment of a depressive symptom, (S)-norketamine, a prodrug of (S)-norketamine, or a pharmacologically acceptable salt of any of these in an amount effective for alleviation of the depressive symptom.

10. The method for prevention and/or treatment of a depressive symptom according to 9 above, wherein the depressive symptom is a depressive symptom in depression, a depressive symptom in obsessive-compulsive disorder, a depressive symptom in PTSD, or a depressive symptom in autism spectrum disorder.

11. The method for prevention and/or treatment of a depressive symptom according to 9 or 10 above, wherein the pharmacologically acceptable salt of (S)-norketamine is (S)-norketamine hydrochloride.

12. The method for prevention and/or treatment of a depressive symptom according to 9 or 10 above, wherein the prodrug of (S)-norketamine is a compound represented by the following formula (VI), (VII), (XIII), or (XIV):

[F9]

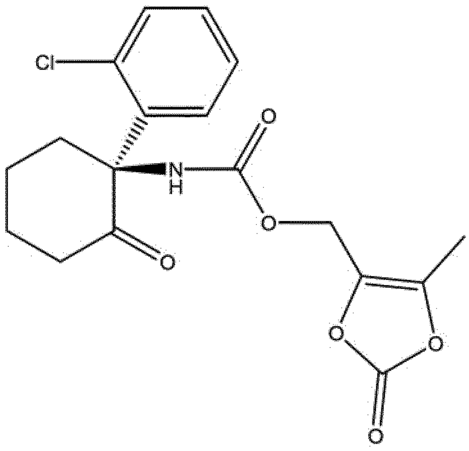

(XIII)

[F10]

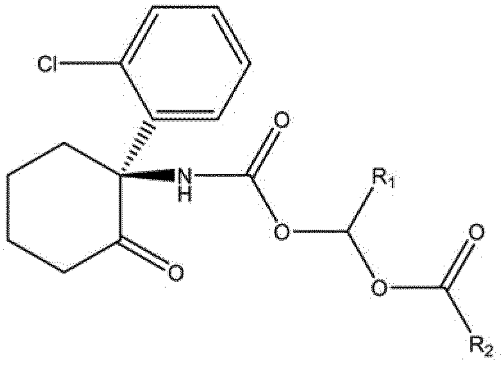

(VI)

[F11]

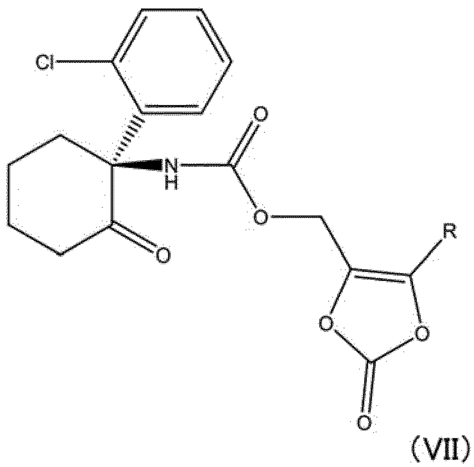

(VII)

[F12]

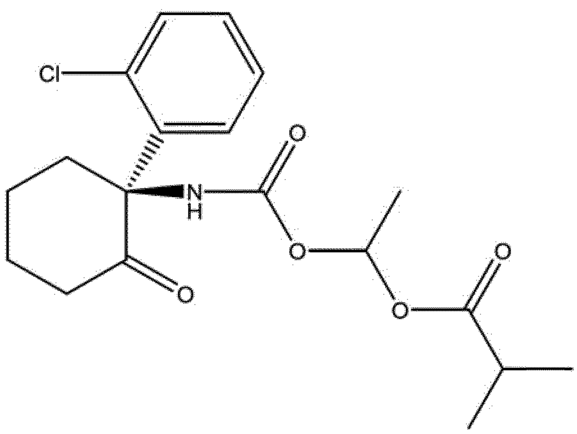

(XIV)

(wherein R represents an alkyl group, an alkoxy group, an aryl group, or an aralkyl group, and $R_1$ and $R_2$ each

independently represent an alkyl group, an alkoxy group, an aryl group, or an aralkyl group), or a pharmacologically acceptable salt or hydrochloride of the compound.

13. Use of (S)-norketamine, a prodrug of (S)-norketamine, or a pharmacologically acceptable salt of any of these for production of a pharmaceutical composition for prevention and/or treatment of a depressive symptom.

14. The use according to 13 above, wherein the depressive symptom is a depressive symptom in depression, a depressive symptom in obsessive-compulsive disorder, a depressive symptom in PTSD, or a depressive symptom in autism spectrum disorder.

15. The use according to 13 or 14 above, wherein the pharmacologically acceptable salt of (S)-norketamine is (S)-norketamine hydrochloride.

16. The use according to 13 or 14 above, wherein the prodrug of (S)-norketamine is any one compound selected from the compounds represented by the following formulae (III) to (XIV):

[F13]

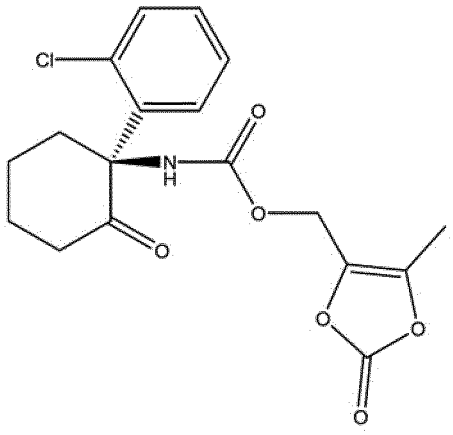

(XIII)

[F14]

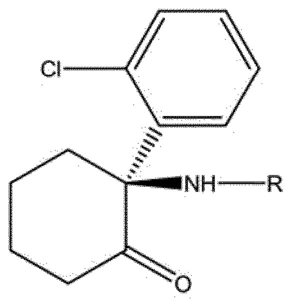

(III)

[F15]

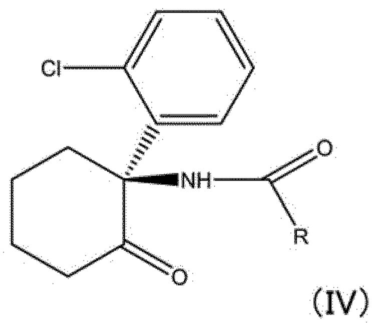

(IV)

[F16]

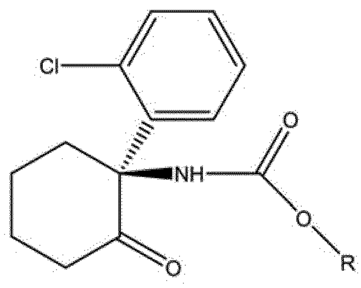

(V)

[F17]

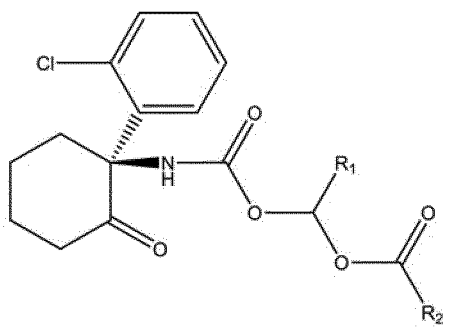

(VI)

[F18]

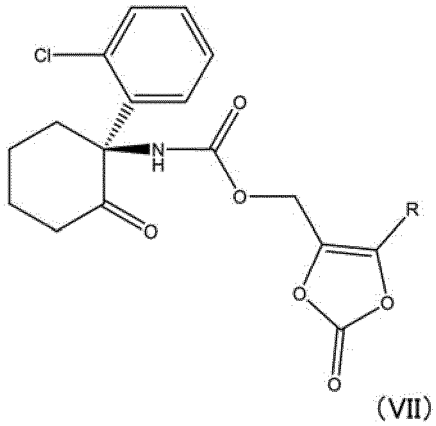

(VII)

[F19]

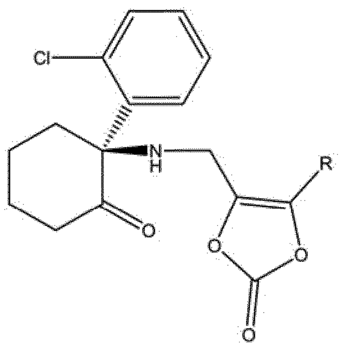

(VIII)

[F20]

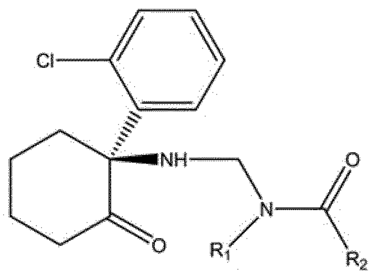

(IX)

[F21]

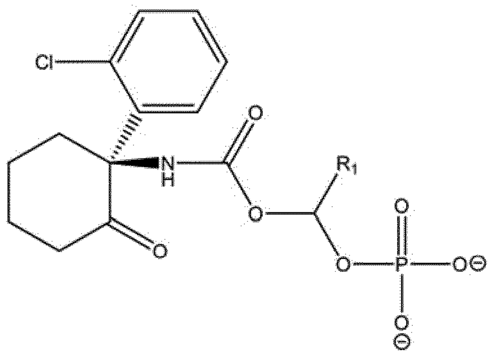

(X)

[F22]

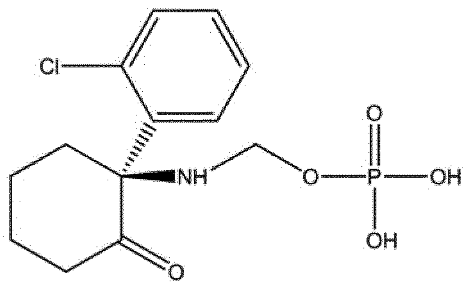

(XI)

[F23]

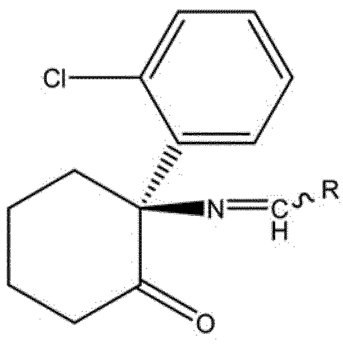

(XII)

[F24]

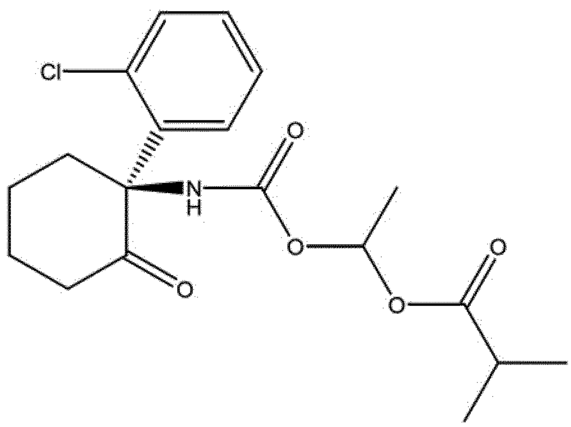

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group), or a pharmacologically acceptable salt or hydrochloride of the compound.

17. (5-Methyl-2-oxo-1,3-dioxol-4-yl)methyl-(S)-(1-(2-chlorophenyl)-2-oxocyclohexyl) carbamate.

18. 1-((((S)-1-(2-Chlorophenyl)-2-oxocyclohexyl)carbamoyl)oxy)ethyl isobutyrate.

19. A drug for prevention and/or treatment of a depressive symptom, the drug containing (5-methyl-2-oxo-1,3-dioxol-4-yl)methyl-(S)-(1-(2-chlorophenyl)-2-oxocyclohexyl) carbamate or 1-((((S)-1-(2-chlorophenyl)-2-oxocyclohexyl)carbamoyl)oxy)ethyl isobutyrate.

20. (S)-Norketamine, a prodrug of (S)-norketamine, or a pharmacologically acceptable salt of any of these for prevention and/or treatment of a depressive symptom.

21. (S)-Norketamine, a prodrug of (S)-norketamine, or a pharmacologically acceptable salt of any of these for prevention and/or treatment of a depressive symptom, which is a compound represented by the formulae (VI), (VII), (XIII), or (XIV) described later wherein R represents an alkyl group, an alkoxy group, an aryl group, or an aralkyl group, and $R_1$ and $R_2$ each independently represent an alkyl group, an alkoxy group, an aryl group, or an aralkyl group.

Effects of the Invention

Norketamine, in particular, (S)-norketamine (i.e., an optical isomer of norketamine), has a long-lasting antidepressant effect and is less likely to exhibit side effects, and thus is effective for the prevention and/or treatment of psychiatric diseases exhibiting depressive symptoms. Therefore, an agent consisting essentially of (S)-norketamine or a pharmacologically acceptable salt thereof, or a pharmaceutical composition containing (S)-norketamine or a pharmacologically acceptable salt thereof and being substantially free of (R)-norketamine or a pharmacologically acceptable salt thereof, is useful as a novel pharmaceutical in the field of prevention and/or treatment of psychiatric diseases exhibiting depressive symptoms.

Brief description of the Drawings

[Fig. 1A] A diagram describing a test protocol for examining the antidepressant effect of norketamine. The test was performed by using, as an inflammatory model of depression, eight-week-old male C57BL/6 mice (purchased from CLEA Japan, Inc.) intraperitoneally administered lipopolysaccharide (hereinafter abbreviated as "LPS") (0.5 mg/kg) (hereinafter the mice will be referred to as "LPS-treated mice"). Saline (10 mL/kg) or norketamine (5, 10, or 20 mg/kg) was intraperitoneally administered 23 hours after administration of LPS. A locomotion test (hereinafter may be abbreviated as "LMT"), a tail suspension test (hereinafter may be abbreviated as "TST"), and a forced swimming test (hereinafter may be abbreviated as "FST") were respectively performed one hour, three hours, and five hours thereafter. (Example 1)

[Fig. 1B] A graph describing the results of the amount of locomotion after administration of norketamine examined by the locomotion test. There was no difference in amount of locomotion between normal mice (Normal), LPS-treated mice administered saline, and LPS-treated mice administered norketamine (5, 10, or 20 mg/kg). The vertical

axis of the graph illustrates the amount of locomotion (counts/60 minutes). (Example 1)

[Fig. 1C] A graph describing the results of the antidepressant effect of norketamine examined by the tail suspension test. LPS-treated mice administered saline exhibited a significant increase in immobility time, as compared with normal mice. Norketamine dose-dependently reduced immobility time, which increased in LPS-treated mice. The vertical axis of the graph illustrates immobility time (seconds) in the TST. (Example 1)

[Fig. 1D] A graph describing the results of the antidepressant effect of norketamine examined by the forced swimming test. LPS-treated mice administered saline exhibited a significant increase in immobility time, as compared with normal mice. Norketamine dose-dependently reduced immobility time, which increased in LPS-treated mice. The vertical axis of the graph illustrating immobility time (seconds) in the FST. (Example 1)

[Fig. 2A] A diagram describing a test protocol for comparing the antidepressant effect of ketamine with that of norketamine. Eight-week-old male C57/BL6 mice (purchased from CLEA Japan, Inc.) were intraperitoneally administered LPS (0.5 mg/kg), and saline (10 mL/kg), ketamine (10 mg/kg), or norketamine (10 mg/kg) was intraperitoneally administered 23 hours thereafter. LMT, TST, and FST were respectively performed one hour, three hours, and five hours after administration. (Example 2)

[Fig. 2B] A graph describing the results of comparison of the amount of locomotion after administration of ketamine and norketamine. There was no difference in amount of locomotion between normal mice, LPS-treated mice administered saline, LPS-treated mice administered ketamine (10 mg/kg), and LPS-treated mice administered norketamine (10 mg/kg). The vertical axis of the graph illustrates the amount of locomotion (counts/60 minutes). (Example 2)

[Fig. 2C] A graph showing the test protocol of comparison between the antidepressant effects of ketamine and norketamine examined by the TST. LPS-treated mice administered saline exhibited a significant increase in immobility time, as compared with normal mice. Ketamine and norketamine significantly reduced immobility time, which increased in LPS-treated mice. The antidepressant effect of norketamine was significantly stronger compared to ketamine. The vertical axis of the graph illustrates immobility time (seconds) in the TST. (Example 2)

[Fig. 2D] A graph describing the test protocol of comparison between the antidepressant effects of ketamine and norketamine examined by the FST. LPS-treated mice administered saline exhibited a significant increase in immobility time, as compared with normal mice. Ketamine and norketamine significantly reduced immobility time, which increased in LPS-treated mice. The antidepressant effect of norketamine was significantly stronger than that of ketamine. The vertical axis of the graph illustrates immobility time (seconds) in the FST. (Example 2)

[Fig. 3A] Illustrates the results of comparison between the side effects of ketamine and norketamine examined by a hyperlocomotion effect (i.e., one basis for evaluation of side effects). Mice administered ketamine (10 mg/kg) or norketamine (20 mg/kg) exhibited a significant increase in amount of locomotion 10 minutes after administration of each drug, as compared with normal mice administered saline. In contrast, administration of norketamine (5 mg/kg or 10 mg/kg) did not affect the amount of locomotion. The vertical axis of the graph illustrates the amount of locomotion (counts/10 minutes). (Example 3)

[FIG. 3B] Illustrates the results of comparison between the side effects of ketamine and norketamine examined by a change in prepulse (PP) inhibition (i.e., one basis for evaluation of side effects). Administration of ketamine (10 mg/kg) induced prepulse inhibition deficit by prepulse stimulation at 77 dB or 81 dB (Fig. 3B). Meanwhile, administration of norketamine (20 mg/kg) significantly induced prepulse inhibition deficit by prepulse stimulation at 81 dB, but administration of norketamine (5 mg/kg or 10 mg/kg) did not induce prepulse inhibition deficit. The vertical axis of the graph illustrates prepulse inhibition (%). (Example 3)

[Fig. 4A] A diagram wherein the antidepressant effects of (S)-norketamine and (R)-norketamine in social defeat stress mice are examined. In the figure, S-NK and R-NK respectively illustrate a group of social defeat stress mice administered (S)-norketamine (10 mg/kg) and a group of social defeat stress mice administered (R)-norketamine (10 mg/kg), Saline a group of social defeat stress mice administered saline (10 mL/kg), and Control a group of normal mice administered saline. In Fig. 4A, LMT represents a locomotion test, TST a tail suspension test, FST a forced swimming test, and SPT a 1% sucrose preference test. (Example 4)

[Fig. 4B] A graph illustrating the results of the antidepressant effect of (S)- or (R)-norketamine in social defeat stress mice examined by the LMT one day after administration of the drug. In the figure, S-NK and R-NK respectively represent a group of social defeat stress mice administered (S)-norketamine and a group of social defeat stress mice administered (R)-norketamine, Saline a group of social defeat stress mice administered saline, and Control a group of normal mice administered saline. The vertical axis of the graph illustrates the amount of locomotion (counts/60 minutes). (Example 4)

[Fig. 4C] A graph illustrating the results of the antidepressant effect of (S)- or (R)-norketamine in social defeat stress mice examined by the TST one day after administration of the drug. In the figure, S-NK and R-NK respectively represent a group of social defeat stress mice administered (S)-norketamine and a group of social defeat stress mice administered (R)-norketamine, Saline a group of social defeat stress mice administered saline, and Control a group of normal mice administered saline. The vertical axis of the graph illustrates immobility time (seconds) in the TST. (Example 4)

[Fig. 4D] A graph illustrating the results of the antidepressant effect of (S)- or (R)-norketamine in social defeat stress mice examined by the FST two days after administration of the drug. In the figure, S-NK and R-NK respectively represent a group of social defeat stress mice administered (S)-norketamine and a group of social defeat stress mice administered (R)-norketamine; Saline represents a group of social defeat stress mice administered saline; and Control represents a group of normal mice administered saline. The vertical axis of the graph illustrates immobility time (seconds) in the FST. (Example 4)

[Fig. 4E] A graph showing the results of the antidepressant effect of (S)- or (R)-norketamine in social defeat stress mice examined by the 1% sucrose preference test seven days after administration of the drug. In Fig. 4E, S-NK and R-NK respectively represent a group of social defeat stress mice administered (S)-norketamine and a group of social defeat stress mice administered (R)-norketamine, Saline a group of social defeat stress mice administered saline, and Control a group of normal mice administered saline. The vertical axis of the graph illustrates sucrose preference (%). (Example 4)

[Fig. 4F] A graph illustrating the results of the effect of (S)- or (R)-norketamine on the spine density of a brain region in social defeat stress mice examined eight days after administration of the drug. (Example 4)

[Fig. 4G] A graph showing the results of comparison between the side effects of (S)-norketamine and (S)-ketamine examined by a hyperlocomotion effect (i.e., one basis for evaluation of side effects). In the figure, S-norket, S-ket, and Saline respectively represent a group of mice administered (S)-norketamine, a group of mice administered (S)-ketamine, and a group of mice administered saline. The vertical axis of the graph illustrates the amount of locomotion (counts/10 minutes). (Example 4)

[Fig. 4H] A graph illustrating the results of comparison between the side effects of (S)-norketamine and (S)-ketamine examined by a prepulse inhibition test (i.e., one basis for evaluation of side effects). In the figure, Saline, S-ket, and S-norket respectively represent a group of mice administered saline, a group of mice administered (S)-ketamine, and a group of mice administered (S)-norketamine. Furthermore, each bar (from left to right) represents a group of mice administered saline, a group of mice administered (S)-ketamine (10 mg/kg), a group of mice administered (S)-norketamine (5 mg/kg), a group of mice administered (S)-norketamine (10 mg/kg), and a group of mice administered (S)-norketamine (20 mg/kg). The vertical axis of the graph illustrates prepulse inhibition (%). (Example 4)

[Fig. 4I] A graph illustrating the results of comparison between the side effects of (S)-norketamine and (S)-ketamine examined by a conditioned place preference test (CPP test) (i.e., one basis for evaluation of side effects). In the figure, S-norket, S-ket, and Saline respectively represent a group of mice administered (S)-norketamine (20 mg/kg), a group of mice administered (S)-ketamine (20 mg/kg), and a group of mice administered saline (10 mL/kg). The vertical axis of the graph illustrates CPP score. (Example 4)

[Fig. 5] Synthesis schemes of (S)-norketamine derivatives. (Example 5)

[Fig. 6A] A diagram illustrating a protocol for examining the antidepressant effect of an (S)-norketamine derivative in social defeat stress mice. In the figure, Vehicle illustrates medium (0.5% carboxymethyl cellulose (CMC) (10 mL/kg) and 0.4% DMSO), and Compound 1 an (S)-norketamine derivative (30 mg/kg). PO means peroral administration. LMT means locomotion test, and SPT a 1% sucrose preference test. The vertical axis of the graph illustrates the amount of locomotion (counts/60 minutes). (Example 7)

[Fig. 6B] A graph illustrating the results of the antidepressant effect of the (S)-norketamine derivative in social defeat stress mice examined by the LMT on the basis of the amount of locomotion one hour after peroral administration of the derivative. In the figure, Vehicle illustrates medium (0.5% carboxymethyl cellulose (CMC) (10 mL/kg) and 0.4% DMSO), and Compound 1 the (S)-norketamine derivative (30 mg/kg). Control represents a group of medium-administered normal mice. The vertical axis of the graph corresponds to the amount of locomotion (counts/60 minutes). (Example 7)

[Fig. 6C] A graph showing the results of the antidepressant effect of the (S)-norketamine derivative in social defeat stress mice examined by the 1% sucrose preference test on the basis of sucrose preference three days and seven days after peroral administration of the derivative. Control illustrates a group of medium-administered normal mice. The vertical axis of the graph corresponds to sucrose preference (%). (Example 7)

## Modes for Carrying Out the Invention

The present invention relates to a drug for the prevention and/or treatment of a depressive symptom, the agent consisting essentially of (S)-norketamine (i.e., an optical isomer of norketamine), a prodrug of (S)-norketamine, or a pharmacologically acceptable salt of any of these. The present invention also relates to a pharmaceutical composition for the prevention and/or treatment of a depressive symptom, the pharmaceutical composition containing (S)-norketamine, a prodrug of (S)-norketamine, or a pharmacologically acceptable salt of any of these in an amount effective for alleviation of the depressive symptom.

In the present invention, it was demonstrated that norketamine (i.e., a major metabolite of ketamine) exhibits a more potent antidepressant effect than ketamine by use of an inflammatory animal model of depression. The animal

model was prepared by the present inventor on the basis of the finding that depression-like behavior was observed in mice administered LPS at an adult stage (Non-Patent Documents 14 to 16).

In the present invention, it was also demonstrated that (S)-norketamine (i.e., a major metabolite of (S)-ketamine) exhibits an antidepressant effect in a social defeat stress model, and the antidepressant effect is observed seven days after administration of (S)-norketamine. It was also demonstrated that a prodrug of (S)-norketamine exhibits an antidepressant effect in the social defeat stress model, and the antidepressant effect is observed seven days after administration of the prodrug.

Norketamine exhibited a more potent antidepressant effect than ketamine in LPS-administered inflammatory model mice of depression through single administration of norketamine (see Examples 1 and 2). (S)-norketamine exhibited a potent antidepressant effect in a social defeat stress model, and the antidepressant effect was observed seven days after administration of (S)-norketamine (see Example 4). Also, a prodrug of (S)-norketamine exhibited a potent antidepressant effect in the social defeat stress model, and the antidepressant effect was observed seven days after administration of the prodrug (see Example 7). Meanwhile, in the case of administration of ketamine, a significant change was observed in hyperlocomotion effect or prepulse inhibition deficit (i.e., one basis for evaluation of side effects). In contrast, such a side effect is reduced in the case of administration of norketamine or (S)-norketamine (see Examples 3 and 4). Also, norketamine, (S)-norketamine, or (R)-norketamine has lower affinity for an NMDA receptor than ketamine (Non-Patent Document 12), and is expected to exhibit less side effects (e.g., psychotomimetic effect) and thus, norketamine, (S)-norketamine, or (R)-norketamine can serve as a promising and safe antidepressant, as compared with ketamine. In fact, ketamine is designated as a scheduled drug, but norketamine is not designated as a scheduled drug.

Similarly to ketamine, norketamine is known to have optical isomers, which are called (R)-norketamine and (S)-norketamine. Ketamine is a racemic mixture containing equal amounts of (R)-ketamine and (S)-ketamine. The present inventors have disclosed that (R)-ketamine or a pharmacologically acceptable salt thereof has a rapid and long-lasting antidepressant effect statistically significantly higher than that of (S)-ketamine and is less likely to exhibit side effects observed in (S)-ketamine, and thus (R)-ketamine or a pharmacologically acceptable salt thereof is effective for the prevention and/or treatment of psychiatric diseases exhibiting depressive symptoms (Patent Documents 1 and 2 and Non-Patent Documents 10 and 11). Among the optical isomers of norketamine, (S)-norketamine was found to exhibit a higher antidepressant effect and less side effects, unlike the case of the optical isomers of ketamine.

(S)-norketamine or a pharmacologically acceptable salt thereof can be used as an antidepressant, specifically, as a drug for the treatment and/or prevention of depressive symptoms (e.g., mood depression, lowering of motivation, anxiety, and accompanying insomnia and anorexia) and suicidal ideation.

The pharmaceutical composition of the present invention may be substantially free of (R)-norketamine or a pharmacologically acceptable salt thereof, and such a pharmaceutical composition is preferred. The term "substantially free of (R)-norketamine or a pharmacologically acceptable salt thereof" refers to the case where (R)-norketamine or a pharmacologically acceptable salt thereof is not contained at all, or (R)-norketamine or a pharmacologically acceptable salt thereof may be contained in such an amount that its effects and side effects are not exhibited, or may be contained as such an impurity that is mixed inevitably during the production of the pharmaceutical composition. For example, the pharmaceutical composition may contain (R)-norketamine or a pharmacologically acceptable salt thereof in an amount of 2 wt% or less, preferably 1 wt% or less, and more preferably 0.5 wt% or less. Furthermore, for example, the pharmaceutical composition (100 mg) may contain (R)-norketamine or a pharmacologically acceptable salt thereof in an amount of 2 mg or less, preferably 1 mg or less, and more preferably 0.5 mg or less. Also, for example, the amount of (R)-norketamine may be 2 mg or less, preferably 1 mg or less, and more preferably 0.5 mg or less, relative to 100 mg of (S)-norketamine contained in the pharmaceutical composition.

Also, the pharmaceutical composition of the present invention may also be substantially free of ketamine, (R)-ketamine, (S)-ketamine, or a pharmacologically acceptable salt of any of these, and such a pharmaceutical composition is preferred. The term "substantially free of ketamine, (R)-ketamine, (S)-ketamine, or a pharmacologically acceptable salt of any of these" refers to the case where ketamine, (R)-ketamine, (S)-ketamine, or a pharmacologically acceptable salt of any of these is not contained at all, or ketamine, (R)-ketamine, (S)-ketamine, or a pharmacologically acceptable salt of any of these may be contained in such an amount that its effects and side effects are not exhibited, or may be contained as such an impurity that is mixed inevitably during the production of the pharmaceutical composition. For example, the pharmaceutical composition may contain ketamine, (R)-ketamine, (S)-ketamine, or a pharmacologically acceptable salt of any of these in an amount of 2 wt% or less, preferably 1 wt% or less, and more preferably 0.5 wt% or less. For example, the pharmaceutical composition (100 mg) may contain ketamine, (R)-ketamine, (S)-ketamine, or a pharmacologically acceptable salt of any of these in an amount of 2 mg or less, preferably 1 mg or less, and more preferably 0.5 mg or less.

The drug or pharmaceutical composition of the present invention is preferably applicable to diseases exhibiting depressive symptoms; for example, depression, MDD, and bipolar disorder involving a repeat of depressive symptoms and manic symptoms as their opposite symptoms. Ketamine has been reported to be effective for treatment-resistant obsessive-compulsive disorder, treatment-resistant PTSD, and autism spectrum disorder (Non-Patent Documents 5 to

7 and 9),and thus, the drug or pharmaceutical composition of the present invention is preferably applicable to obsessive-compulsive disorder, PTSD, and autism spectrum disorder. Obsessive-compulsive disorder, which is a type of anxiety disorder, is a disease with pathological conditions characterized by obsessions and compulsions, and is considered to be associated with depression, and there are extremely many cases wherein patients with obsessive-compulsive disorder have depression as well and exhibit depressive symptoms in addition to obsessions and compulsions. Patients with PTSD exhibit depressive symptoms in many cases, and in fact, an antidepressant (e.g., SSRI) is used as a therapeutic drug for PTSD, but its therapeutic effects are weak. Autism spectrum disorder is a type of developmental disorder involving, for example, inability to maintain a normal social relationship with others, abnormal use of language, and actions such as menacing behavior. The present invention encompasses a pharmaceutical composition for the prevention and/or treatment of obsessive-compulsive disorder, PTSD, or autism spectrum disorder, the pharmaceutical composition containing (S)-norketamine or a pharmacologically acceptable salt thereof in an amount effective for alleviation of the symptoms of obsessive-compulsive disorder, PTSD, or autism spectrum disorder.

The drug or pharmaceutical composition of the present invention can be administered perorally or parenterally. The peroral administration may use a known dosage form for administration, such as a tablet, a capsule, a coated tablet, a troche, or a liquid (e.g., a solution or a suspension). Furthermore, examples of the parenteral administration include intravenous, intramuscular, or subcutaneous administration by injection; transmucosal administration via the nasal cavity or the oral cavity by use of, for example, a spray or an aerosol; rectal administration by use of, for example, a suppository; and transdermal administration by use of, for example, a patch, a liniment, or a gel. Preferable examples are peroral administration, transnasal administration, and intravenous administration by injection.

(S)-Norketamine is a compound represented by the following formula (I) and can be used in the form of a free base or a pharmacologically acceptable salt thereof. The pharmacologically acceptable salt is preferably a pharmacologically acceptable acid addition salt, and more preferably a hydrochloride.

[F25]

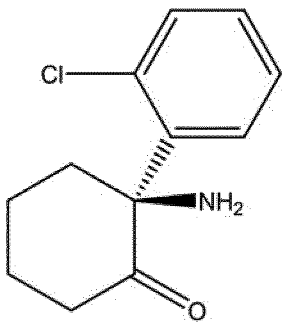

(I)

(S)-Norketamine can be produced by any known method. For example, (S)-norketamine can be produced from 1-(2-chlorophenyl)-1-cyclohexene (Non-Patent Document 17, the following formula (II)).

[F26]

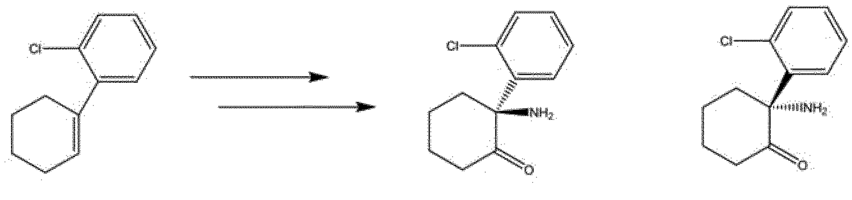

(S)-Norketamine (R)-Norketamine

(II)

(S)-Norketamine or a pharmacologically acceptable salt thereof may be subjected to a modification; for example, substitution of the chlorine (i.e., a substituent) by another halogen (fluoride, bromide, iodide), to thereby produce a derivative, and there is a possibility that a compound having more preferred effects may be obtained.

Furthermore, a prodrug of (S)-norketamine or a pharmacologically acceptable salt thereof may be synthesized

and developed as a pharmaceutical. Prodrug refers to a compound which itself exhibits no or little pharmacological effect of interest, but, after being administered in vivo, is transformed into an active metabolite through in vivo metabolism and exhibits a desired pharmacological effect. That is, a prodrug of (S)-norketamine means a compound which itself exhibits no or little pharmacological effect of interest, but, after being administered in vivo, is transformed into (S)-norketamine through in vivo metabolism and exhibits the effect of alleviating depressive symptoms. The prodrug of (S)-norketamine may be designed by any known, reported method (Non-Patent Documents 18 and 19). The prodrug of (S)-norketamine is not particularly limited, so long as it is a compound which is transformed into (S)-norketamine through in vivo metabolism and exhibits the effect of alleviating depressive symptoms, and for example, examples can include a compound prepared through introduction of a substituent to the nitrogen atom of the amino group of (S)-norketamine. Specific examples of the prodrug of (S)-norketamine include N-alkylated (S)-norketamine represented by the formula (III) described below; N-amides of (S)-norketamine represented by the formula (IV) described below; N-carbamates of (S)-norketamine represented by the formula (V) described below; N-acyloxyalkyl carbamates of (S)-norketamine represented by the formula (VI) described below; oxodioxolenylmethyl carbamates of (S)-norketamine represented by the formula (VII) described below; N-oxodioxolenylmethyl derivatives of (S)-norketamine represented by the formula (VIII) described below; N-Mannich bases of (S)-norketamine represented by the formula (IX) described below; phosphoryloxymethyl carbamates of (S)-norketamine represented by the formula (X) described below; N-phosphates of (S)-norketamine represented by the formula (XI) described below; and imines of (S)-norketamine represented by the formula (XII) described below. In the below-described formulae including R, the substituent R is preferably an alkyl group, an alkoxy group, an aryl group, or an aralkyl group, more preferably a lower alkyl group or a lower alkoxy group, and still more preferably a methyl group, an ethyl group, a butyl group, a methoxy group, an ethoxy group, or a butoxy group. In the below-described formulae including $R_1$ and $R_2$, the substituents $R_1$ and $R_2$ is each independently preferably an alkyl group, an alkoxy group, an aryl group, or an aralkyl group, more preferably a lower alkyl group or a lower alkoxy group, and still more preferably a methyl group, an ethyl group, a propyl group, an isopropyl group, a butyl group, a methoxy group, an ethoxy group, or a butoxy group. Examples of the compound satisfying R, $R_1$, and $R_2$ include (5-methyl-2-oxo-1,3-dioxol-4-yl)methyl-(S)-(1-(2-chlorophenyl)-2-oxocyclohexyl) carbamate represented by the formula (XIII) described below, and 1-((((S)-1-(2-chlorophenyl)-2-oxocyclohexyl)carbamoyl)oxy)ethyl isobutyrate represented by the formula (XIV) described below.

A pharmacologically acceptable salt of prodrug of (S)-norketamine may be used in the form of a free base or a pharmacologically acceptable salt thereof. The pharmacologically acceptable salt is preferably a pharmacologically acceptable acid addition salt, and more preferably a hydrochloride.

[F27]

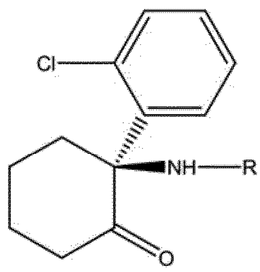

(III)

[F28]

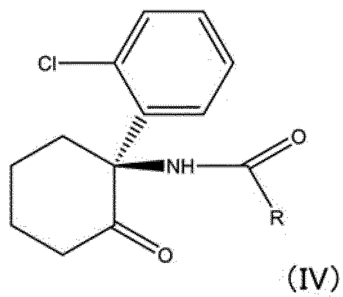

(IV)

[F29]

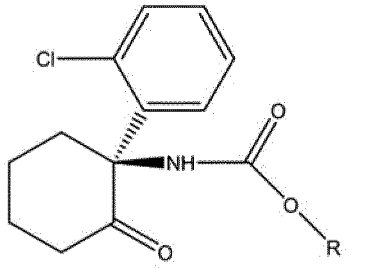

(V)

[F30]

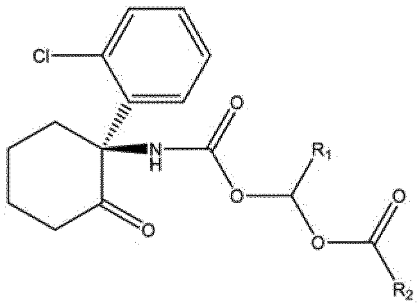

(VI)

[F31]

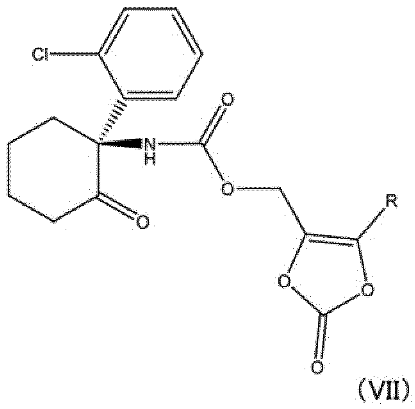

(VII)

[F32]

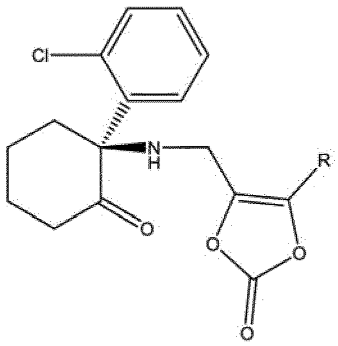

(VIII)

[F33]

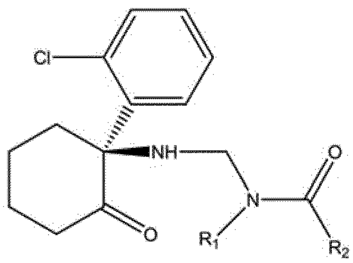

(IX)

[F34]

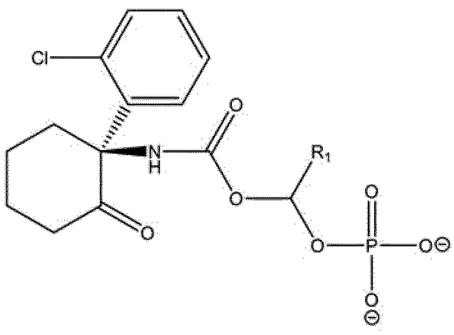

(X)

[F35]

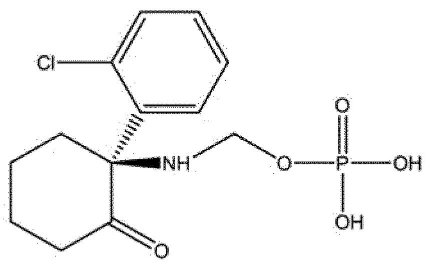

(XI)

[F36]

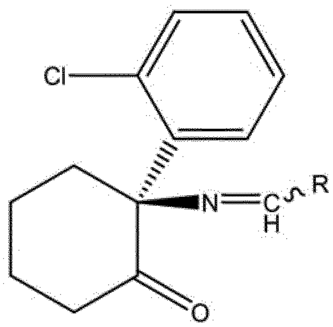

(XII)

[F37]

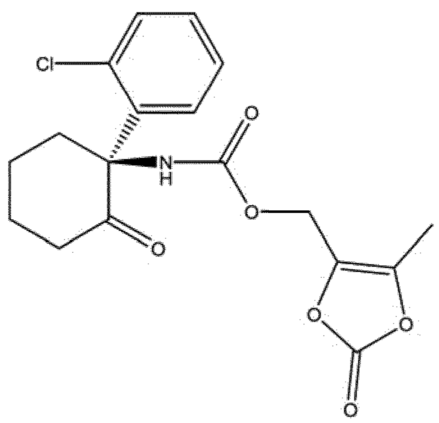

(XIII)

[F38]

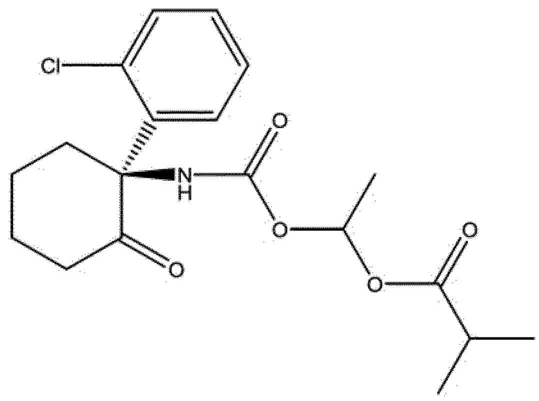

(XIV)

Also, by labelling the compound of the present invention with an isotope (e.g., a stable isotope $^{13}C$ or $^{2}H(D)$), the compound can be quantitatively determined for its in vivo kinetics and the like.

The pharmaceutical composition of the present invention may contain a pharmaceutically active ingredient effective for depressive symptoms in addition to (S)-norketamine, a prodrug of (S)-norketamine, or a pharmacologically acceptable salt of any of these. Furthermore, the pharmaceutical composition may appropriately contain, in addition to such a pharmaceutically active ingredient, a suitable pharmacologically acceptable carrier well known to those skilled in the art, depending on the dosage form or the like. Examples of the pharmacologically acceptable carrier may include an antioxidant, a stabilizer, a preservative, a flavoring agent, a colorant, a solvent, a solubilizer, a surfactant, an emulsifier, an antifoaming agent, a viscosity adjustor, a gelling agent, an absorption accelerator, a dispersant, an excipient, and a pH adjustor.

When the drug or pharmaceutical composition of the present invention is prepared as a formulation for injection, it is preferably in the form of a solution or a suspension, and when it is for transmucosal administration via the nasal cavity, the oral cavity, or the like, it is preferably in the form of a powder, a drop, or an aerosol. Furthermore, when it is for rectal administration, it is preferably in the form of a semi-solid formulation, such as a cream or a suppository. Each of these formulations can be prepared by any of the methods known to those skilled in the art of pharmacy as described in, for example, Remington's Pharmaceutical Sciences (Mack Publishing Company, Easton, PA, 1970). The formulation for injection may contain, as a carrier, a plasma-derived protein (e.g., albumin), an amino acid (e.g., glycine), or a sugar (e.g., mannitol), and may further contain, for example, a buffer, a solubilizing aid, or an isotonic agent. Furthermore, when used as a watersoluble formulation or a lyophilized formulation, it is preferable to add a surfactant (e.g., Tween™ 80 or Tween™ 20) for the prevention of aggregation. Also, a dosage form for parenteral administration other than the formulation for injection may contain distilled water, saline, polyalkylene glycol (e.g., polyethylene glycol), a plant-derived oil, hydrogenated naphthalene, or the like. For example, a formulation for rectal administration (e.g., a suppository) contains a common excipient, such as polyalkylene glycol, petroleum jelly, or cacao oil. A vaginal formulation may contain an absorption accelerator, such as a bile salt, an ethylenediamine salt, and a citric acid salt. A formulation for inhalation may be in a solid form, or may contain an excipient, such as lactose, and additionally, a transnasal drop may be an aqueous or oil solution.

The accurate dosage and dosing regimen of the drug or pharmaceutical composition of the present invention can be determined depending on the required amounts, treatment methods, diseases, degrees of necessity for individual treatment targets, or the like. The dosage can be specifically determined depending on the age, body weight, general health condition, sex, meal, administration time, administration method, excretion rate, drug combination, pathological condition of a patient of interest, or the like, and additionally, the dosage may be determined in consideration of other factors. When the pharmaceutical composition of the present invention is administered for a disease exhibiting a depressive symptom (e.g., depression, bipolar disorder, or obsessive-compulsive disorder), the pharmaceutical composition preferably contains an active ingredient in an amount effective for alleviation of a symptom of the disease (e.g., depression, bipolar disorder, or obsessive-compulsive disorder), preferably a depressive symptom of each disease. (S)-Norketamine or a pharmacologically acceptable salt thereof can be safely used since it is less likely to exhibit side effects observed in ketamine, and the daily dose thereof may vary depending on, the condition or body weight of a patient, the type of a compound, an administration route, or the like, and in the case of, for example, parenteral administration, the dosage (in terms of the amount of an active ingredient) is about 0.01 to about 1,000 mg/person/day, preferably 0.1 to 500 mg/person/day, 0.1 to 100 mg/person/day, 1.0 to 100 mg/person/day, 10 to 100 mg/person/day,

100 to 200 mg/person/day, 200 to 300 mg/person/day, 300 to 400 mg/person/day, or 400 to 500 mg/person/day, and furthermore, in the case of peroral administration, the dosage (in terms of the amount of an active ingredient) is about 0.01 to about 500 mg/person/day, preferably 0.1 to 100 mg/person/day, 0.1 to 1.0 mg/person/day, 1.0 to 20 mg/person/day, 20 to 40 mg/person/day, 40 to 60 mg/person/day, or 80 to 100 mg/person/day.

The present invention also relates to a method comprising administering the drug or pharmaceutical composition of the present invention to a patient in need of prevention and/or treatment of a depressive symptom. Furthermore, the present invention relates to a method for the prevention and/or treatment of a depressive symptom, the method comprising administering the drug of the present invention to a subject in need thereof in an amount effective for alleviation of the depressive symptom. The present invention also relates to a method for the prevention and/or treatment of a depressive symptom, the method comprising administering the pharmaceutical composition of the present invention to a subject in need thereof in an amount effective for alleviation of the depressive symptom. The subject of administration may be a human or mammal diagnosed as having a depressive symptom, or a human or mammal requiring alleviation of a depressive symptom.

Furthermore, the present invention relates to use of (S)-norketamine, a prodrug of (S)-norketamine, or a pharmacologically acceptable salt of any of these for production of a pharmaceutical composition for the prevention and/or treatment of a depressive symptom.

Furthermore, the present invention relates to (S)-norketamine, a prodrug of (S)-norketamine, or a pharmacologically acceptable salt of any of these for the prevention and/or treatment of a depressive symptom.

The present invention will be described below in more detail by way of examples, but the present invention is not limited to these examples. Furthermore, various modifications of the present invention may be made within a scope not departing from the technical concept of the invention. All tests were performed under the approval of the Animal Care and Use Committee of Chiba University.

Example 1

An inflammatory animal model of depression (Non-Patent Documents 14 to 16) was used to examine the antidepressant effect of norketamine (i.e., a major metabolite of ketamine) on the depression-like behavior of the model animal.

1. Materials and methods

Norketamine hydrochloride was purchased from Tocris Bioscience (Bristol, UK). Saline was used as a negative control of the drug.

The inflammatory animal model of depression was prepared by administering lipopolysaccharide (hereinafter abbreviated as "LPS") to adult mice. Depression-like behavior was observed in the LPS-administered mice, and in light of this, it was suggested that the mice can be used as a new animal model of depression. The model mice were prepared by the present inventor and his collaborators, which has been reported in several papers (Non-Patent Documents 14 to 16). As compared with normal mice, the model mice exhibited an increase in immobility time in both a tail suspension test (hereinafter abbreviated as "TST") and a forced swimming test (hereinafter abbreviated as "FST"), each of which is a behavioral test used as an indicator for screening of antidepressants. In contrast, there was no difference in amount of locomotion between the LPS-administered mice and the normal mice in a locomotion test (hereinafter abbreviated as "LMT"), which is used as an indicator of motor function. In light of these results, it was suggested that depression-like behavior was induced in the model mice through administration of LPS.

The antidepressant effect of norketamine was examined by the behavioral tests LMT, TST, and FST, all in adult mice. Fig. 1A illustrates the administration schedules of LPS and norketamine. The TST and the FST were both performed after administration of norketamine. The TST was performed as follows. Firstly, the mice were taken out from a cage, and then a small piece of an adhesive tape was pasted onto a portion about 2 cm from the tip of their tails. A small hole was opened in the small piece, and each mouse was hung on a hook. The immobility time of each mouse was recorded for 10 minutes. Mice were considered immobile only when they hung passively and completely motionless. The immobility time increases in a depressive state. The FST was performed as follows. Firstly, each mouse was placed in a cylinder (diameter: 23 cm, height: 31 cm) containing water (height: 15 cm) and maintained at 23 $\pm$ 1°C. The mice were tested in an automated forced-swimming apparatus using a SCANET MV-40 (MELQUEST Ltd., Toyama, Japan). The immobility time (i.e., a value obtained by subtracting active time from total time) was calculated using analytical software of the apparatus. Cumulative immobility time was recorded over six minutes during the test. The LMT was performed as follows. Firstly, the mice were placed in experimental cages (length $\times$ width $\times$ height: 560 mm $\times$ 560 mm $\times$ 330 mm). The locomotor activity of the mice was counted by SCANET MV-40, and the cumulative exercise of the mouse was observed for 60 minutes. The cage was cleaned between a test and the next test. The immobility time increases in a depressive state.

Statistical analysis was performed by one-way analysis of variance (one-way ANOVA) and a subsequent least significant difference test (LSD test). Data are represented as the mean ± standard error (n = 8 to 12 mice/group). Significant differences as compared with a LPS-treated mouse group administered saline are indicated by *p<0.05, **p<0.01, and ***p<0.001. Significant differences as compared with a LPS-treated mouse group administered ketamine are indicated by $^{\#}$p<0.05 and $^{\#\#}$p<0.01.

2. Results

In the LMT, there was no difference in amount of locomotion between the normal mice, the LPS-treated mice administered saline, and the LPS-treated mice administered norketamine (5, 10, or 20 mg/kg) (Fig. 1B). Thus, these treatments were found not to affect motor function.

In contrast, in the TST and the FST, a significant increase in immobility time was observed in the LPS-treated mice administered saline, as compared with the normal mice. Norketamine dose-dependently reduced immobility time, which increased in the LPS-treated mice (Figs. 1C and 1D).

In light of these results, it was found that norketamine exhibits an antidepressant effect on the LPS-administered mice. That is, the TST and the FST demonstrated the antidepressant effect of norketamine.

Example 2

The antidepressant effect of norketamine was examined in comparison with the antidepressant effect of ketamine. Specifically, the inflammatory animal model of depression (Non-Patent Documents 14 to 16) was used to examine the antidepressant effects of ketamine and norketamine on the depression-like behavior of the model animal.

1. Materials and methods

Norketamine hydrochloride was purchased from Tocris Bioscience (Bristol, UK). Ketamine hydrochloride (Ketalar(TM)) was purchased from Daiichi Sankyo Company, Limited (Tokyo, Japan). Saline was used as a negative control of the drug.

In the same manner as in Example 1, an inflammatory animal model of depression was prepared by administering lipopolysaccharide (hereinafter abbreviated as "LPS") to adult mice.

With the same method as the method described in Example 1, the antidepressant effects of ketamine and norketamine on adult mice were examined by the behavioral tests LMT, TST, and FST. Fig. 2A illustrates the administration schedules of LPS, ketamine, and norketamine. Statistical analysis was performed with the same method as the method described in Example 1.

2. Results

In the LMT, there was no difference in amount of locomotion between the normal mice, the LPS-treated mice administered saline, and the LPS-treated mice administered ketamine (10 mg/kg) or norketamine (10 mg/kg) (Fig. 2B). Thus, these treatments were found not to affect motor function.

In contrast, in the TST and the FST, a significant increase in immobility time was observed in the LPS-treated mice administered saline, as compared with the normal mice. Both ketamine and norketamine significantly reduced immobility time, which increased in the LPS-treated mice (Figs. 2C and 2D). The antidepressant effect of norketamine was significantly stronger compared to ketamine (Figs. 2C and 2D) .

In light of these results, it was found that ketamine and norketamine (dose: 10 mg/kg) exhibit an antidepressant effect on the LPS-administered mice. Notably, the antidepressant effect of norketamine was significantly stronger than that of ketamine. These results indicate that norketamine has a more potent antidepressant effect than ketamine. Since norketamine has weaker affinity for an NMDA receptor than ketamine, it is thought that the antidepressant effect of norketamine is attributed to a factor other than blocking of the NMDA receptor.

Example 3

The side effects of ketamine and norketamine were compared by a hyperlocomotion test and a prepulse inhibition test (i.e., bases for evaluation of side effects).

1. Materials and methods

The effect of ketamine or norketamine on the amount of locomotion of mice was tested using SCANET MV-40

(MELQUEST Ltd., Toyama, Japan). Specifically, the amount of locomotion was measured for a total of 180 minutes (i.e., from 60 minutes before administration to 120 minutes after administration), and calculated as an amount of locomotion per 10 minutes. Statistical analysis of the results of the amount of locomotion was performed by repeated one-way analysis of variance (repeated one-way ANOVA) and then a subsequent least significant difference test (LSD test). Data are represented as the mean ± standard error (n = 7 or 8 mice/group). Significant differences as compared with a group administered saline are indicated by $^{**}p<0.05$ and $^{***}p<0.001$. Significant differences as compared with a group administered ketamine (10 mg/kg) are indicated by $^{\#\#}p<0.01$.

The prepulse inhibition test was performed using a startle response system (SR-LAB, San Diego Instruments, San Diego, CA, USA). Specifically, mice were habituated to a sound of 65 dB (i.e., background noise) in the apparatus, and thereafter, the mice were given an auditory stimulation at 69, 73, 77, or 81 dB (prepulse stimulation) for more than 20 milliseconds, and 100 milliseconds thereafter, the mice were given an auditory stimulation at 120 dB (pulse stimulation), and a startle response to the pulse stimulation was recorded. A startle response to the pulse stimulation without the prepulse stimulation was also recorded. Then, PPI was calculated on the basis of the resultant data by use of the following formula:

$$PPI(\%) = [1 - (pPx/P120)] \times 100$$

In this formula, PPI means prepulse inhibition, pPx indicates the maximum intensity of startle to the pulse stimulation with the prepulse stimulation, and P120 indicates the average of the maximum intensities of startle to the pulse stimulation without the prepulse stimulation. Analysis of the results of prepulse inhibition was performed by Wilks' lambda, which is a multivariate analysis of variance, and a subsequent least significant difference test (LSD test). Data are represented as the mean ± standard error (n = 10 to 12 mice/group). Significant differences as compared with a group administered saline are indicated by $^{*}p<0.05$ and $^{***}p<0.001$.

2. Results

In the measurement of the amount of locomotion, a significant increase in amount of locomotion was observed in the mice administered ketamine (10 mg/kg) 10 minutes after administration of the drug, as compared with the normal mice administered saline. A significant increase in amount of locomotion was observed in the mice administered norketamine (20 mg/kg) 10 minutes after administration of the drug, as compared with the normal mice administered saline. Furthermore, the amount of locomotion 10 minutes after administration of norketamine (20 mg/kg) was significantly lower than that in the mice administered ketamine (10 mg/kg). The amount of locomotion was transiently enhanced after administration of ketamine or norketamine and returned to a normal value 20 minutes after administration of the drug. In contrast, the administration of norketamine (5 mg/kg or 10 mg/kg) did not affect the amount of locomotion (Fig. 3A).

In the prepulse inhibition test, the administration of ketamine (10 mg/kg) induced prepulse inhibition deficit by a prepulse stimulation at 77 dB or 81 dB (Fig. 3B). In contrast, the administration of norketamine (20 mg/kg) significantly induced prepulse inhibition deficit by a prepulse stimulation at 81 dB, but the administration of norketamine (5 mg/kg or 10 mg/kg) did not induce prepulse inhibition deficit (Fig. 3B).

As described above, the administration of ketamine was found to induce side effects, such as hyperlocomotion effect, prepulse inhibition deficit, and drug dependence, but hyperlocomotion effect and prepulse inhibition deficit of the administration of norketamine was lower than ketamine. Thus, norketamine is a drug safer than ketamine.

Example 4

A social defeat stress model of depression (Non-Patent Document 11) was used to examine the antidepressant effect of (S)- or (R)-norketamine on the depression-like behavior of the model animal. Furthermore, the side effects of (S)-norketamine were examined.

1. Materials and methods

(S)- or (R)-Norketamine hydrochloride was prepared from norketamine via optical resolution. The purity of each these isomers was found using high-performance liquid chromatography (CHIRALPAK(TM) IA, column size: 250 × 4.6 mm, mobile phase: n-hexane/dichloromethane/diethylamine (75/25/0.1), Daicel Corporation, Tokyo, Japan). (S)-Ketamine used as a control for examination of the side effects was prepared by a previously reported method (Patent Documents 1 to 3).

A social defeat stress model of depression was produced by bringing C57BL/6 male mice into contact with ICR

male mice (large aggressive mice) for 10 consecutive days to apply a stress called a "social defeat stress" to the C57BL/6 male mice according to a previous report (Non-Patent Document 11). Depression-like behavior was observed in the mice that had received the social defeat stress. Specifically, an increase in immobility time was observed in the social defeat stress model in each of the TST and the FST. Furthermore, in a 1% sucrose preference test (SPT), the amount of drinking of sucrose water decreased significantly, thus induction of depression-like behavior was suggested. In contrast, in the LMT, there was no difference in amount of locomotion between the social defeat stress mice and the normal mice.

The production of the model animal of depression and the administration of the drug were specifically performed as described below (Fig. 4A). Male C57BL/6 mice (seven weeks old, Japan SLC, Inc., Hamamatsu, Japan) and ICR mice (nine weeks old, Japan SLC, Inc., Hamamatsu, Japan) were used. The mice were given water and feed ad libitum. A social defeat stress was applied by housing one C57/B6 mouse with one ICR mouse for 10 days. On day 11, a social interaction test was performed to select mice exhibiting depressive symptoms, and they were used in the following behavioral evaluation. Control mice were intraperitoneally administered medium (saline: 10 mL/kg), and the mice exhibiting depressive symptoms were intraperitoneally administered (S)- or (R)-norketamine (10 mg/kg) or medium (saline: 10 mL/kg).

The antidepressant effect of the drug was examined by behavioral tests, such as the TST, the FST, the LMT, and the SPT (Fig. 4A). The LMT and the TST were performed on the day of administration, and the FST was performed on the day after administration. The SPT was performed seven days after administration. The TST was performed as follows. Firstly, the mice taken out from a cage, and then a small piece of an adhesive tape was pasted onto a portion about 2 cm from the tip of their tails. A small hole was opened in the small piece, and each mouse was hung on a hook. The immobility time of each mouse was recorded for 10 minutes. Mice were considered immobile only when they hung passively and completely motionless. The immobility time increases in a depressive state. The FST was performed as follows.

Firstly, each mouse was placed in a cylinder (diameter: 23 cm, height: 31 cm) containing water (height: 15 cm) and maintained at 23 ± 1°C. The mice were tested in an automated forced-swimming apparatus using a SCANET MV-40 (MELQUEST Ltd., Toyama, Japan). The immobility time (i.e., a value obtained by subtracting active time from total time) was calculated by use of the analytical software of the apparatus. Cumulative immobility time was recorded over six minutes during the test. The LMT was performed as follows. Firstly, the mice were placed in experimental cages (length × width × height: 560 mm × 560 mm × 330 mm). The locomotor activity of the mouse was counted by SCANET MV-40, and the cumulative exercise of the mouse was recorded for 60 minutes. The cage was cleaned between a test and the next test. The immobility time increases in a depressive state. The SPT was performed as follows. Mice were given common drinking water and a 1% sucrose solution ad libitum, and the amount of consumption of the sucrose solution was measured. The consumption of the sucrose solution, which is a reward response, decreases in a depressive state. The mice were decapitated eight days after administration of (S)- or (R)-norketamine, and the brain was quickly dissected out and then subjected to Golgi staining. Spine density was quantitatively determined through observation under a KEYENCE microscope (BZ-9000, Osaka, Japan).

The side effects of (S)-norketamine were examined by use of normal mice through a hyperlocomotion test, a prepulse inhibition test, and a conditioned place preference test (CPP) (these tests are bases for evaluation of side effects). For comparison, (S)-ketamine was used as a control for examination of the side effects. For the hyperlocomotion test, the effect of (S)-norketamine or (S)-ketamine on the amount of locomotion of mice was tested using a SCANET MV-40 (MELQUEST Ltd., Toyama, Japan). Measurement was performed for a total of 180 minutes (i.e., 60 minutes before administration to 120 minutes after administration). The amount of locomotion was calculated per 10 minutes. The prepulse inhibition test was performed using a startle response system (SR-LAB, San Diego Instruments, San Diego, CA, USA). Furthermore, the conditioned place preference test was performed using a conditioned place preference test apparatus (Brain Science Idea Co., Ltd., Osaka, Japan).

Statistical analysis of the results of the social defeat stress model was performed by one-way analysis of variance and a subsequent least significant difference test. Data are represented as the mean ± standard error (n = 8 or 9 mice/group). Significant differences as compared with a group of social defeat stress mice administered saline are represented by $^{*}p<0.05$, $^{**}p<0.01$, and $^{***}p<0.001$. Significant differences as compared with a group of social defeat stress mice administered (R)-norketamine are indicated by $^{\#}p<0.05$ and $^{\#\#}p<0.01$.

Statistical analysis of the results of Golgi staining was performed by repeated one-way analysis of variance and a subsequent least significant difference test. Data are represented as the mean ± standard error (n = 6 mice/group). Significant differences as compared with a group of social defeat stress mice administered saline are indicated by $^{**}p<0.01$ and $^{***}p<0.001$. Significant differences as compared with a group of social defeat stress mice administered (R)-norketamine are indicated by $^{\#}p<0.05$ and $^{\#\#\#}p<0.001$.

Statistical analysis of the results of the amount of locomotion was performed by repeated one-way analysis of variance and a subsequent least significant difference test. Data are represented as the mean ± standard error (n = 10 to 12 mice/group). Significant differences as compared with a group of mice administered saline are indicated by $^{**}p<0.01$ and $^{***}p<0.001$.

Analysis of the results of prepulse inhibition was performed by multivariate analysis of variance (MANOVA) and a subsequent least significant difference test. Data are represented as the mean ± standard error (n = 8 or 9 mice/group). Significant differences as compared with a group of mice administered saline are indicated by **p<0.01.

Analysis of the results of the conditioned place preference test was performed by one-way analysis of variance and a subsequent least significant difference test. Data are represented as the mean ± standard error (n = 7 or 9 mice/group). Significant differences as compared with a group of mice administered saline are represented by *p<0.05 and **p<0.01.

2. Results

In the social defeat stress mice, a significant increase in immobility time was observed in the TST and the FST, and furthermore, a significant reduction in sucrose consumption preference was observed in the SPT, as compared with the normal mice. In contrast, in the LMT, there was no difference in amount of locomotion between the social defeat stress mice and the normal mice.

In the LMT performed after administration of both norketamine isomers, there was no difference in amount of locomotion between the normal mice, the social defeat stress mice administered saline, and the social defeat stress mice administered (S)- or (R)-norketamine (Fig. 4B). Thus, these treatments were found not to affect motor function.

In the TST performed after administration of both norketamine isomers, a significant increase in immobility time was observed in the social defeat stress mice administered saline, as compared with the normal mice. (S)-Norketamine significantly reduced immobility time in the social defeat stress mice in the TST, but (R)-norketamine did not exhibit an antidepressant effect (Fig. 4C). (S)-Norketamine exhibited a significantly higher antidepressant effect than (R)-norketamine (Fig. 4C).

In the FST performed after administration of both norketamine isomers, a significant increase in immobility time was observed in the social defeat stress mice administered saline, as compared with the normal mice. (S)-Norketamine significantly reduced immobility time in the social defeat stress mice in the TST {FST}, but (R)-norketamine did not exhibit an antidepressant effect (Fig. 4D). (S)-Norketamine exhibited a significantly higher antidepressant effect than (R)-norketamine (Fig. 4D).

In the SPT performed seven days after administration of both norketamine isomers, a reduction in sucrose consumption preference was observed in the social defeat stress mice administered saline, as compared with the normal mice. (S)-Norketamine significantly restored the sucrose consumption preference, which reduced in the social defeat stress mice seven days after administration of the drug, but (R)-norketamine did not restore it. The difference between (S)-norketamine and (R)-norketamine was found to be statistically significant (Fig. 4E).

In the Golgi staining performed eight days after administration of both norketamine isomers, the social defeat stress mice administered saline exhibited a significant decrease in spine density in the frontal cortex (prelimbic region), the hippocampus CA3 region, and the hippocampal dentate gyrus, as compared with the normal mice. (S)-Norketamine significantly increased the spine density, which decreased in the social defeat stress mice eight days after administration of the drug, but (R)-norketamine did not exhibit the effect of increasing the spine density (Fig. 4F). In contrast, there was no change in spine density in the hippocampus CA1 region of the social defeat stress mice. Furthermore, the spine density increased in the nucleus accumbens (core region and shell region) of the social defeat stress mice, but was not affected by administration of (S)-norketamine or (R)-norketamine.

Next, in the hyperlocomotion test for examination of the side effects, a significant increase in amount of locomotion was observed in the mice administered (S)-ketamine (10 mg/kg) 10 minutes and 20 minutes after administration of the drug, as compared with the normal mice administered saline. The amount of locomotion was transiently enhanced after administration of (S)-ketamine (10 mg/kg) and returned to a normal value 30 minutes after administration of the drug. In contrast, the administration of (S)-norketamine (5, 10, or 20 mg/kg) did not affect the amount of locomotion (Fig. 4G).

In the prepulse inhibition test after administration of (S)-ketamine or (S)-norketamine, the administration of (S)-ketamine (10 mg/kg) induced prepulse inhibition deficit (Fig. 4H). In contrast, the administration of (S)-norketamine (5, 10, or 20 mg/kg) did not induce prepulse inhibition deficit (Fig. 4H).

In the conditioned place preference test after administration of (S)-ketamine or (S)-norketamine, the administration of (S)-ketamine (20 mg/kg) was found to significantly increase the CPP score and to induce drug dependence (Fig. 4I). In contrast, the administration of (S)-norketamine (20 mg/kg) did not increase the CPP score (Fig. 4I).

In light of the aforementioned results, it was found that (S)-norketamine (dose: 10 mg/kg) exhibited an antidepressant effect in the social defeat stress mice, but (R)-norketamine did not exhibit an antidepressant effect. Notably, (S)-norketamine exhibited a significantly stronger antidepressant effect than (R)-norketamine in the SPT, the TST, and the FST. These results indicate that (S)-norketamine has a more long-lasting antidepressant effect than (R)-norketamine. Both isomers of ketamine or its metabolite are known to exhibit a rapid in vivo clearance. (S)-Norketamine exhibited an antidepressant effect, although it is considered to be eliminated from the body seven days after its single administration.

This indicates that the difference in antidepressant effect between both isomers of norketamine seven days after administration thereof is not attributed to a difference in pharmacokinetics.

Also, from the viewpoint of side effects, a hyperlocomotion effect, induced prepulse inhibition deficit, and drug dependence were found by the administration of (S)-ketamine. In contrast, the administration of (S)-norketamine did not exhibit a hyperlocomotion effect and did not induce prepulse inhibition deficit or drug dependence. In light of the results, (S)-norketamine is a drug safer than (RS)-ketamine and (S)-ketamine, which are clinically used at present.

Example 5

(S)-Norketamine derivatives were synthesized through procedures described below. Fig. 5 illustrates synthesis schemes. Compounds (1) to (9) described below correspond to 1 to 9 in Fig. 5.

1. Synthesis of compound (2)

Compound (1) (i.e., cyclohexanone) (3.09 g, 31.4 mmol) and 1-bromo-2-chlorobenzene (3.00 g, 15.7 mmol) were added to a mixture of $Pd_2(dba)_3$ (48 mg, 0.053 mmol), Xantphos (73 mg, 0.13 mmol), cesium carbonate (7.61 g, 23.3 mmol), and 1,4-dioxane (11 mL) in a nitrogen atmosphere, and the resultant mixture was heated with agitation at 100°C for 20 hours. The mixture was left to cool, and water and ethyl acetate were added to the mixture, followed by extraction and phase separation. The aqueous layer was subjected to extraction with ethyl acetate, and the mixed organic layer was washed with saturated brine, followed by drying over anhydrous magnesium sulfate. The resultant product was concentrated under reduced pressure, and the resultant residue was subjected to silica gel chromatographic purification (neutral $SiO_2$ (65 g), elution with a mixture of hexane and ethyl acetate (gradient from 20:1 to 10:1), to thereby produce compound (2) (1.93 g, 9.26 mmol) in the form of a white solid (yield: 59.0%).

The synthesis of compound (2) was confirmed through NMR.

2. Synthesis of compound (3)

tert-Butyl azodicarboxylate (1.90 g, 8.23 mmol), powdery MS5A (1.06 g), and (R)-$C_8$-TCYP (0.76 g, 0.633 mmol) were added to a solution (6.3 mL) of compound (2) (1.32 g, 6.33 mmol) in dichloromethane solution in a nitrogen atmosphere, and the resultant mixture was heated with agitation at 45°C for two hours in an open system, to thereby remove dichloromethane. The resultant residue was heated in a nitrogen atmosphere at 45°C for 60 hours. The residue was left to cool and then subjected to silica gel chromatographic purification (neutral $SiO_2$ (56 g), hexane/ethyl acetate = 9:1), to thereby produce compound (3) (2.50 g, 5.70 mmol) (yield: 90.0%).

The synthesis of compound (3) was confirmed through NMR.

3. Synthesis of (S)-norketamine

Trifluoroacetic acid (25 mL) was added to a solution (49 mL) of compound (3) (2.50 g, 5.70 mmol) in dichloromethane solution in a nitrogen atmosphere, and the resultant mixture was agitated at room temperature for three hours. Acetone (29 mL) was added to the mixture, and it was agitated for 10 minutes and then concentrated under reduced pressure. A solvent mixture of acetic acid-THF-water (3:1:1 v/v/v) (46 mL) was added to the resultant residue, and then zinc powder (9.12 g, 140 mmol) was added thereto in several portions. The resultant mixture was agitated at room temperature for 30 minutes, and then heated with agitation at 60°C for four hours. The mixture was left to cool, and then diluted with dichloromethane, and saturated aqueous sodium carbonate solution was added to the mixture, followed by extraction and phase separation. The aqueous layer was subjected to extraction with dichloromethane five times, and the mixed organic layer was dried over anhydrous sodium sulfate. The resultant product was concentrated under reduced pressure, and the resultant residue was subjected to silica gel chromatographic purification (neutral $SiO_2$ (25 g), hexane/ethyl acetate = 1:2), to thereby produce (S)-norketamine (1.00 g, 4.47 mmol) in the form of a white solid (yield: 78.4%) .

The synthesis of (S)-norketamine was confirmed through NMR.

The S-isomer was detected 100% by chiral chromatography, and the R-isomer was not detected. The retention time corresponded to that of the S-isomer prepared through optical resolution of a racemic mixture with D-tartaric acid [Nature, 533, 481 (2016)].

4. Synthesis of compound (5)

N,N'-disuccinimidyl carbonate (0.83 g, 3.23 mmol) was added to a solution (7.3 mL) of compound (4) (0.35 g, 2.69 mmol) and triethylamine (0.33 g, 3.23 mmol) in acetonitrile solution in a nitrogen atmosphere, and the resultant

mixture was agitated at room temperature for three hours. Dichloromethane (73 mL) and water were added to the mixture, and then 1N hydrochloric acid (3.23 mL, 3.23 mmol) was added thereto, followed by extraction and phase separation. The organic layer was sequentially washed with water, saturated aqueous sodium hydrogen carbonate solution, and water, and then dried over anhydrous magnesium sulfate. The resultant product was concentrated under reduced pressure, and the resultant residue was subjected to silica gel chromatographic purification (neutral $SiO_2$ (29 g), hexane/ethyl acetate = 1:1), to thereby produce compound (5) (0.19 g, 0.701 mmol) in the form of a colorless oily product (yield: 26.1%).
The synthesis of compound (5) was confirmed through NMR.

5. Synthesis of (S)-norketamine derivative (1)

(S)-Norketamine (0.16 g, 0.716 mmol) and saturated aqueous sodium hydrogen carbonate solution (0.95 mL) were added to a solution (1.9 mL) of compound (5) (0.19 g, 0.701 mmol) in acetonitrile solution in a nitrogen atmosphere, and the resultant mixture was agitated at room temperature overnight. Ethyl acetate and water were added to the mixture, followed by extraction and phase separation. The organic layer was dried over anhydrous sodium sulfate, and then concentrated under reduced pressure, and the resultant residue was subjected to silica gel chromatographic purification (neutral $SiO_2$ (11 g), hexane/ethyl acetate = 2:1), to thereby produce (S)-norketamine derivative (1) represented by the following formula (XIII) (0.16 g, 0.422 mmol) in the form of a colorless resin (yield: 60.2%).
The synthesis of (S)-norketamine derivative (1) was confirmed through NMR.

[F39]

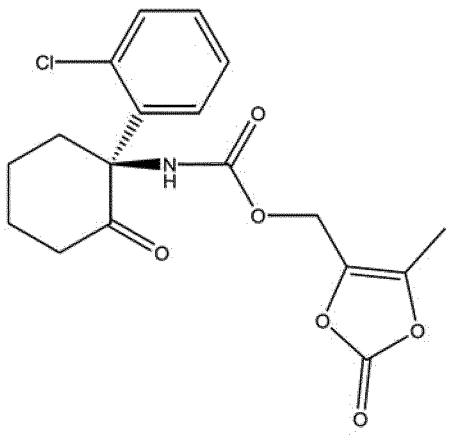

(XIII)

6. Synthesis of compound (7)

15% Aqueous MeSNa solution (13.6 g, 29.1 mmol) was added dropwise to a mixture of compound (6) (4.17 g, 29.2 mmol), n-Bu4N·HSO4 (0.10 g, 0.292 mmol), and dichloromethane (11 mL) over two hours in a nitrogen atmosphere. Also, the resultant mixture was agitated at room temperature for one hour, to thereby separate an organic layer. The organic layer was washed twice with saturated brine and dried over anhydrous sodium sulfate. The resultant product was concentrated under reduced pressure, and the resultant residue was distilled (boiling point: 173°C, ambient pressure), to thereby produce compound (7) (3.35 g, 21.6 mmol) as a colorless oily product (yield: 74.2%).
The synthesis of compound (7) was confirmed through NMR.

7. Synthesis of compound (8)

A mixture of compound (7) (3.35 g, 21.6 mmol) and isobutyric acid (2.85 g, 32.3 mmol) was added dropwise to a mixture of isobutyric acid (2.85 g, 32.3 mmol) and diisopropylethylamine (4.18 g, 32.4 mmol) in a nitrogen atmosphere. After completion of dropwise addition, the resultant mixture was heated with agitation at 55°C for 16 hours. The mixture was left to cool and then diluted with diethyl ether (220 mL). The diluted product was washed with water four times, with saturated aqueous sodium hydrogen carbonate solution twice, and with saturated brine once, and then dried over anhydrous magnesium sulfate. The resultant product was concentrated under reduced pressure, and the resultant residue was distilled under reduced pressure (boiling point: 84 to 91°C/6 mmHg), to thereby produce compound (8) (4.33 g, 21.0 mmol) in the form of a colorless oily product (yield: 97.0%).

The synthesis of compound (8) was confirmed through NMR.

8. Synthesis of compound (9)

N-Hydroxysuccinimide (1.68 g, 14.6 mmol) was added to a solution (35 mL) of compound (8) (1.52 g, 7.38 mmol) in dichloromethane solution in a nitrogen atmosphere, and the resultant mixture was cooled in an ice bath. 9% Peracetic acid/acetic acid solution (12.3 g, 14.6 mmol) was added dropwise to the mixture over 10 minutes, and the mixture was agitated at room temperature for 24 hours. The mixture was diluted with diethyl ether (180 mL) and washed with water twice, with saturated aqueous sodium hydrogen carbonate solution three times (until weakly basic pH), and with saturated brine once, and dried over anhydrous magnesium sulfate. The resultant product was concentrated under reduced pressure, and the resultant residue was subjected to silica gel chromatographic purification (neutral $SiO_2$ (40 g), hexane/ethyl acetate = 3:1), to thereby produce compound (9) (1.20 g, 4.40 mmol) in the form of a colorless oily product (yield: 59.6%).

The synthesis of compound (9) was confirmed through NMR.

9. Synthesis of (S)-norketamine derivative (2)

(S)-Norketamine (0.14 g, 0.626 mmol) and saturated aqueous sodium hydrogen carbonate solution (0.85 mL) were added to a solution (1.7 mL) of compound (5) (0.17 g, 0.626 mmol) in acetonitrile solution in a nitrogen atmosphere, and the resultant mixture was agitated at room temperature overnight. Ethyl acetate and water were added to the mixture, followed by extraction and phase separation. The organic layer was dried over anhydrous sodium sulfate, and then concentrated under reduced pressure, and the resultant residue was subjected to silica gel chromatographic purification (neutral $SiO_2$ (10 g), hexane/ethyl acetate = 5:1), to thereby produce (S)-norketamine derivative (2) represented by the following formula (XIV) (0.18 g, 0.471 mmol) in the form of a colorless oily product of diastereomeric mixture (yield: 75.2%).

The synthesis of (S)-norketamine derivative (2) was confirmed through NMR.

[F40]

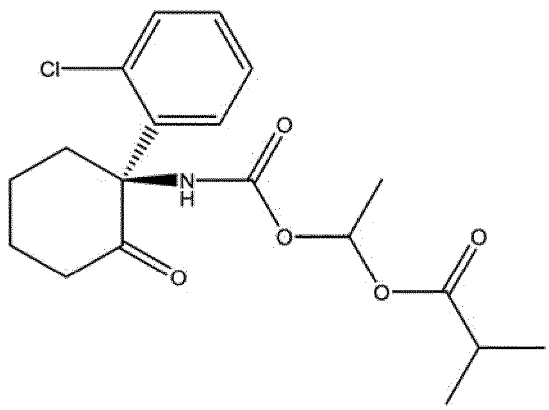

(XIV)

Example 6

The pharmacokinetic profile of an (S)-norketamine derivative was investigated through measurement of the blood concentration of (S)-norketamine after administration thereof.

1. Materials and methods

Male C57/B6 mice (seven to eight weeks old, Japan SLC, Inc., Hamamatsu, Japan) were used. The mice were given water and feed ad libitum. A suspension of an (S)-norketamine derivative ((S)-norketamine derivative (1) or (S)-norketamine derivative (2)) (30 mg/kg) in medium (0.5% carboxymethyl cellulose (CMC) 10 mL/kg, 0.4% DMSO) was perorally administered to each mouse, and 15 minutes, 30 minutes, one hour, two hours, four hours, and eight hours after the administration, the mouse was anesthetized with 5% isoflurane, then blood was collected from the heart and placed into a tube containing EDTA, and blood plasma was obtained through centrifugation. The obtained blood plasma was placed into a polypropylene microtube and stored in a freezer at -80°C.

The plasma concentration of (S)-norketamine was measured by the method described below.
STD (standard) solution (10 μL) was added to mouse plasma (20 μL) as a calibration curve sample. A mixture of acetonitrile/water (1:1, v/v) was added to a sample, a blank sample, and a QC (quality control) sample.
Agitation was performed for 10 seconds, and I.S. (internal standard) solution (Norketamine-$D_4$: 10 ng/mL) (100 μL) was added. A mixture of acetonitrile and methanol (= 9:1) was added to the blank sample.
Agitation was performed for 30 seconds, followed by centrifugation at room temperature and 16,000 × g for three minutes. The supernatant (80 μL) was transferred to a new microtube, and 0.001 mol/L ammonium hydrogen carbonate (100 μL) was added to the microtube.
Agitation was performed for 10 seconds, followed by centrifugation at room temperature and 16,000 × g for two minutes.
The (S)-norketamine concentration was measured through HPLC.
The measurement conditions are as follows.

• LC conditions

High-performance liquid chromatograph: LC-20A system (Shimadzu Corporation)
Column for analysis: CHIRALPAK AS-3R, 3 μm, 4.6 mm × 100 mm, DAICEL
Column temperature: 25°C
Mobile phase: 0.001 mol/L ammonium hydrogen carbonate/acetonitrile (54:46, v/v)
Flow rate: 1.0 mL/min
Autosampler cleaning liquid: 0.001 mol/L ammonium hydrogen carbonate/acetonitrile (54:46, v/v)
Autosampler temperature: 4°C

• MS/MS conditions

Tandem Mass Spectrometer: API5000 (AB Sciex Pte. Ltd.)
API interface: Turbo Spray (ESI)
Heated gas temperature: 650°C
Ion-spray voltage: 5,500 V
Nebulizer gas setting (GS1): 60 psi, air
Heated gas setting (GS2): 80 psi, air
Curtain gas setting: 20 psi, nitrogen
Collision gas setting: 4, nitrogen
Ionization mode: MRM mode, positive ion detection mode
Monitored ions and collision energy:
S-Norketamine: m/z 224.1 to m/z 125.1
IS (Norketamine-D4): m/z 228.2 to m/z 129.1

2. Results

Table 1 shows the results of concentration measurement.

[Table 1]

| Compound | Animal No. | Sampling time points | Area Ratio | Calculated Concentration (ng/mL) |
|---|---|---|---|---|
| | A-1 | 15 min | 7.73 | 193 |
| | A-2 | | 4.21 | 105 |
| | A-3 | | 4.28 | 107 |
| | B-1 | 30 min | 7.49 | 187 |
| | B-2 | | 2.26 | 56.4 |
| | B-3 | | 3.46 | 86.3 |
| | C-1 | 1 h | 3.48 | 86.9 |
| | C-2 | | 2.92 | 72.8 |

(continued)

| Compound | Animal No. | Sampling time points | Area Ratio | Calculated Concentration (ng/mL) |
|---|---|---|---|---|
| 1 | C-3 | | 2.94 | 73.3 |
| | D-1 | 2 h | 2.02 | 50.4 |
| | D-2 | | 2.06 | 51.5 |
| | D-3 | | 1.32 | 33.0 |
| | E-1 | 4 h | 0.944 | 23.6 |
| | E-2 | | 0.686 | 17.1 |
| | E-3 | | 0.709 | 17.7 |
| | F-1 | 8 h | 0.140 | 3.48 |
| | F-2 | | 0.0994 | 2.47 |
| | F-3 | | 0.249 | 6.19 |
| 2 | A-1 | 15 min | 3.07 | 76.7 |
| | A-2 | | 2.31 | 57.7 |
| | A-3 | | 3.22 | 80.5 |
| | B-1 | 30 min | 2.18 | 54.4 |
| | B-2 | | 2.26 | 56.5 |
| | B-3 | | 1.28 | 31.9 |
| | C-1 | 1 h | 0.752 | 18.8 |
| | C-2 | | 0.656 | 16.4 |
| | C-3 | | 0.479 | 11.9 |
| | D-1 | 2 h | 0.140 | 3.49 |
| | D-2 | | 0.224 | 5.58 |
| | D-3 | | 0.245 | 6.11 |
| | E-1 | 4 h | 0.212 | 5.28 |
| | E-2 | | 0.138 | 3.43 |
| | E-3 | | 0.104 | 2.57 |
| | F-1 | 8 h | 0.0193 | 0.467 |
| | F-2 | | 0.0165 | 0.398 |
| | F-3 | | 0.0328 | 0.804 |

In Table 1, "compound 1" indicates (S)-norketamine derivative (1), and "compound 2" indicates (S)-norketamine derivative (2). In light of the fact that from the results of Table 1, the blood concentration of (S)-norketamine derivative (1) decreased more slowly than that of (S)-norketamine derivative (2), (S)-norketamine derivative (1) remained in blood for a longer period of time, and thus, (S)-norketamine derivative (1) was selected for the following pharmacological experiment.

Example 7

A social defeat stress model of depression (Non-Patent Document 11) was used to examine the antidepressant effect of (S)-norketamine derivative (1) on the depression-like behavior of the model animal.

1. Materials and methods

A social defeat stress model of depression was prepared by bringing C57BL/6 male mice into contact with ICR male mice (large aggressive mice) for 10 consecutive days to apply a stress called a "social defeat stress" according to a previous report (Non-Patent Document 11). Depression-like behavior was observed in the mice that had received the social defeat stress. Specifically, the social defeat stress model exhibited a significant decrease in amount of drinking of sucrose water in a 1% sucrose preference test (SPT), thus induction of depression-like behavior (anhedonia) was suggested. In contrast, there was no difference in amount of locomotion between the social defeat stress mice and the normal mice.

The preparation of the model animal of depression and the administration of the drug were specifically performed as described below (Fig. 6A). Male C57BL/6 mice (seven weeks old, Japan SLC, Inc., Hamamatsu, Japan) and ICR mice (nine weeks old, Japan SLC, Inc., Hamamatsu, Japan) were used. The mice were given water and feed ad libitum. A social defeat stress was applied by housing one C57BL/6 mouse with one ICR mouse for 10 days. On day 11, a social interaction test was performed to select mice exhibiting depressive symptoms, and they were used for the subsequent behavioral evaluation. Control mice were perorally administered medium (0.5% carboxymethyl cellulose (CMC) 10 mL/kg, 0.4% DMSO), and the mice exhibiting depressive symptoms were perorally administered (S)-norketamine derivative (compound 1) (30 mg/kg) or medium (0.5% carboxymethyl cellulose (CMC) 10 mL/kg, 0.4% DMSO).

The antidepressant effect of the drug was examined by behavioral tests, such as the LMT and the SPT (Fig. 6A). The LMT was performed one hour after the administration, and the SPT was performed two days and six days after the administration. The SPT was performed by giving mice common drinking water and a 1% sucrose solution ad libitum, and the amount of consumption of the sucrose solution was measured. The consumption of the sucrose solution, which is a reward response, decreases in a depressive state.

Statistical analysis was performed by one-way analysis of variance (one-way ANOVA) and a subsequent least significant difference test (LSD test). Data are represented as the mean ± standard error (n = 9 to 11 mice/group). Significant differences as compared with a group of mice exhibiting depressive symptoms and perorally administered (S)-norketamine derivative (1) are indicated by ***$p<0.001$.

2. Results

There was no difference in amount of locomotion between the three groups one hour after the peroral administration (Fig. 6B). In the 1% sucrose preference test three days and seven days after the administration, a significant reduction in consumption of the sucrose solution was observed in the group of mice exhibiting depressive symptoms, but a significant improvement was observed in the group of mice administered compound I (Fig. 6C). These results indicated that compound I has an antidepressant effect in the social defeat stress model.

Industrial Applicability

As described above, the drug or pharmaceutical composition of the present invention for the prevention and/or treatment of depressive symptoms has a rapid and long-lasting antidepressant effect, and furthermore, is less likely to exhibit side effects, such as psychotomimetic effect and drug dependence, and thus, the drug or the pharmaceutical composition is useful as a novel pharmaceutical in the field of the prevention and/or treatment of psychiatric diseases exhibiting depressive symptoms.

## Claims

**1.** An agent for prevention and/or treatment of a depressive symptom, the agent consisting essentially of (S)-norketamine, a prodrug of (S)-norketamine, or a pharmacologically acceptable salt of any of these.

**2.** The agent for prevention and/or treatment of a depressive symptom according to claim 1, wherein the depressive symptom is a depressive symptom in depression, a depressive symptom in obsessive-compulsive disorder, a depressive symptom in posttraumatic stress disorder (PTSD), or a depressive symptom in autism spectrum disorder.

**3.** The agent for prevention and/or treatment of a depressive symptom according to claim 1 or 2, wherein the pharmacologically acceptable salt of (S)-norketamine is (S)-norketamine hydrochloride.

**4.** The agent for prevention and/or treatment of a depressive symptom according to claim 1 or 2, wherein the prodrug of (S)-norketamine is a compound represented by the following formula (VI), (VII), (XIII), or (XIV):

[F1]

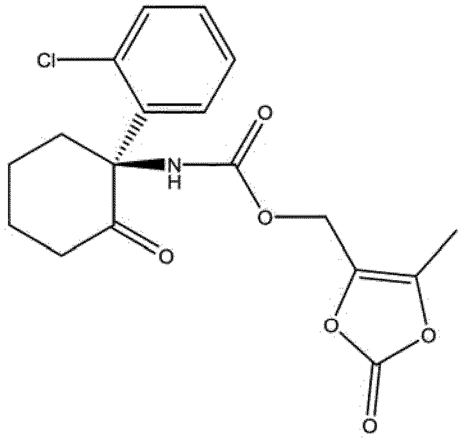

(XIII)

[F2]

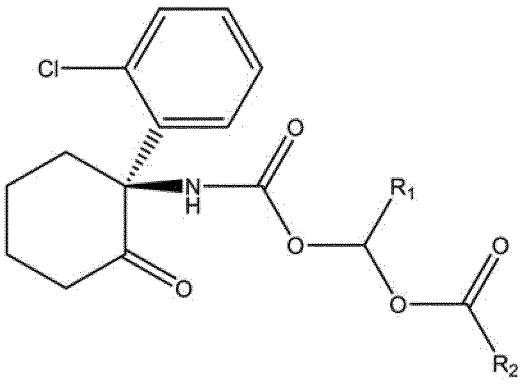

(VI)

[F3]

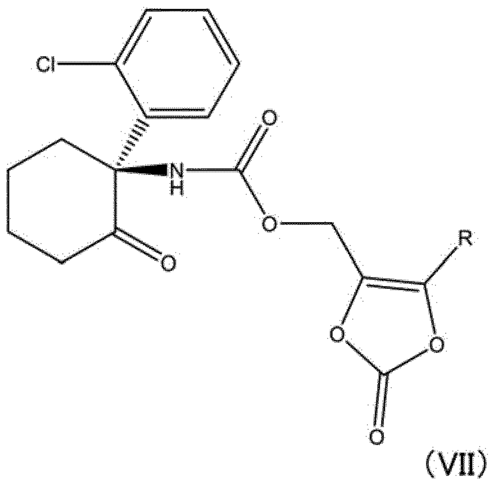

(VII)

[F4]

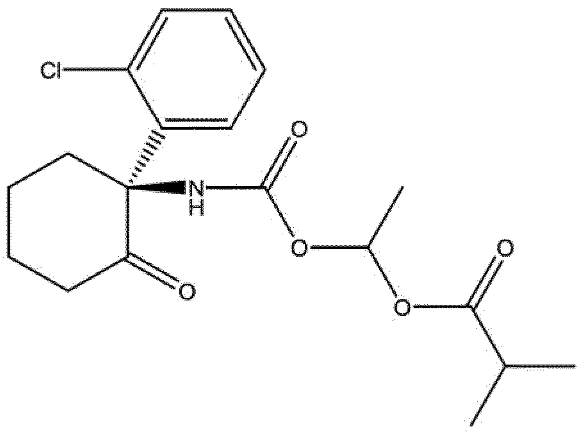

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group), or a pharmacologically acceptable salt or hydrochloride of the compound.

**5.** A pharmaceutical composition for prevention and/or treatment of a depressive symptom, the pharmaceutical composition comprising (S)-norketamine, a prodrug of (S)-norketamine, or a pharmacologically acceptable salt of any of these in an amount effective for alleviation of the depressive symptom, and being substantially free of (R)-norketamine or a pharmacologically acceptable salt thereof.

**6.** The pharmaceutical composition for prevention and/or treatment of a depressive symptom according to claim 5, wherein the depressive symptom is a depressive symptom in depression, a depressive symptom in obsessive-compulsive disorder, a depressive symptom in PTSD, or a depressive symptom in autism spectrum disorder.

**7.** The pharmaceutical composition for prevention and/or treatment of a depressive symptom according to claim 5 or 6, wherein the pharmacologically acceptable salt of (S)-norketamine is (S)-norketamine hydrochloride.

**8.** The pharmaceutical composition for prevention and/or treatment of a depressive symptom according to claim 5 or 6, wherein the prodrug of (S)-norketamine is a compound represented by the following formula (VI), (VII), (XIII), or (XIV):

[F5]

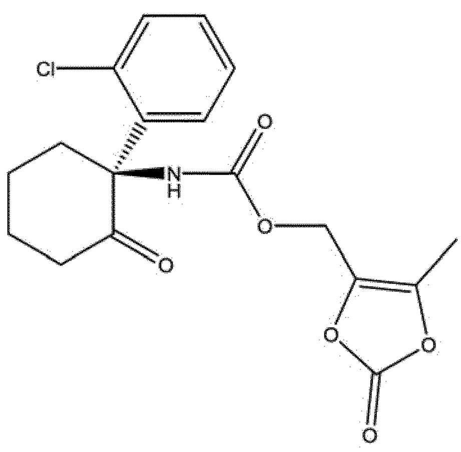

(XIII)

[F6]

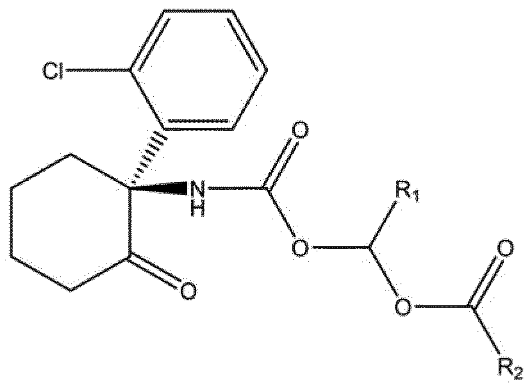

(VI)

[F7]

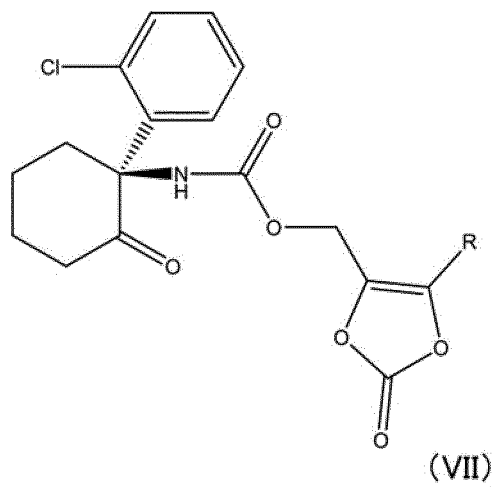

(VII)

[F8]

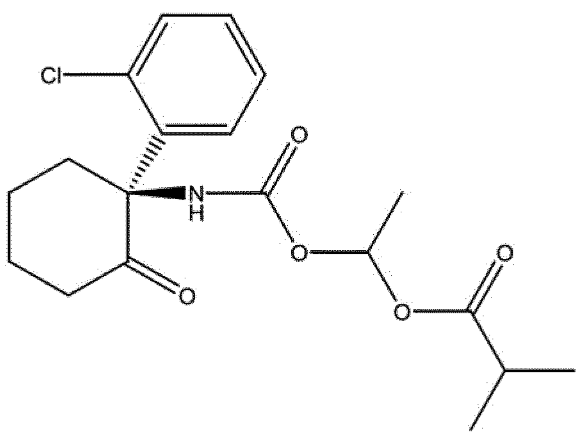

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group), or a pharmacologically acceptable salt or hydrochloride of the compound.

**9.** A method for prevention and/or treatment of a depressive symptom, the method comprising administering, to a patient in need of prevention and/or treatment of a depressive symptom, (S)-norketamine, a prodrug of (S)-norketamine, or a pharmacologically acceptable salt of any of these in an amount effective for alleviation of the depressive symptom.

**10.** The method for prevention and/or treatment of a depressive symptom according to claim 9, wherein the depressive symptom is a depressive symptom in depression, a depressive symptom in obsessive-compulsive disorder, a depressive symptom in PTSD, or a depressive symptom in autism spectrum disorder.

**11.** The method for prevention and/or treatment of a depressive symptom according to claim 9 or 10, wherein the pharmacologically acceptable salt of (S)-norketamine is (S)-norketamine hydrochloride.

**12.** The method for prevention and/or treatment of a depressive symptom according to claim 9 or 10, wherein the prodrug of (S)-norketamine is a compound represented by the following formula (VI), (VII), (XIII), or (XIV):

[F9]

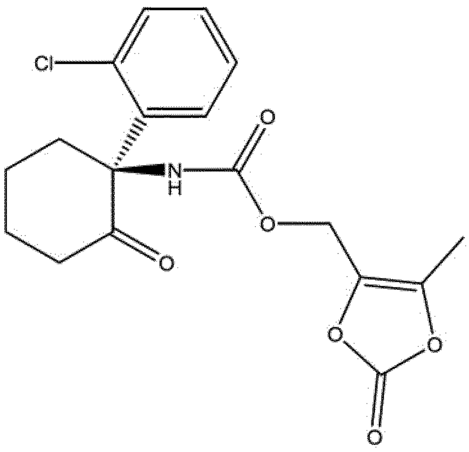

(XIII)

[F10]

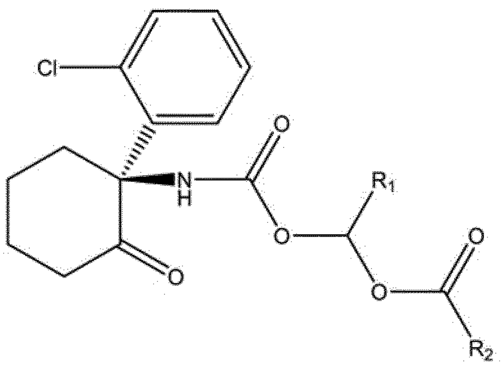

(VI)

[F11]

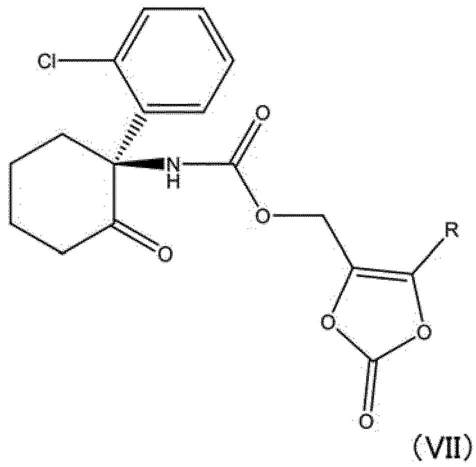

(VII)

[F12]

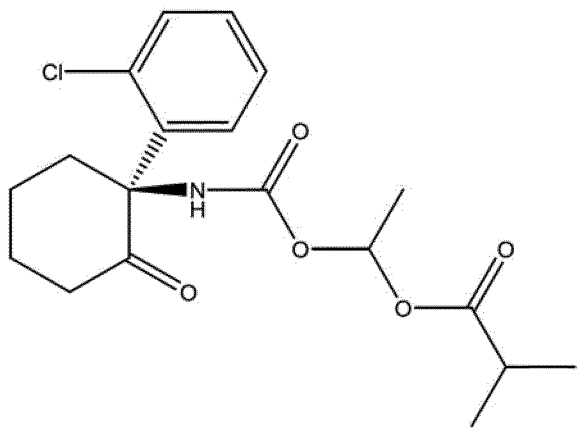

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group), or a pharmacologically acceptable salt or hydrochloride of the compound.

**13.** Use of (S)-norketamine, a prodrug of (S)-norketamine, or a pharmacologically acceptable salt of any of these for production of a pharmaceutical composition for prevention and/or treatment of a depressive symptom.

**14.** Use according to claim 13, wherein the depressive symptom is a depressive symptom in depression, a depressive symptom in obsessive-compulsive disorder, a depressive symptom in PTSD, or a depressive symptom in autism spectrum disorder.

**15.** The use according to claim 13 or 14, wherein the pharmacologically acceptable salt of (S)-norketamine is (S)-norketamine hydrochloride.

**16.** The use according to claim 13 or 14, wherein the prodrug of (S)-norketamine is any one compound selected from the compounds represented by the following formulae (III) to (XIV) or a pharmacologically acceptable salt or hydrochloride of the compound:

[F13]

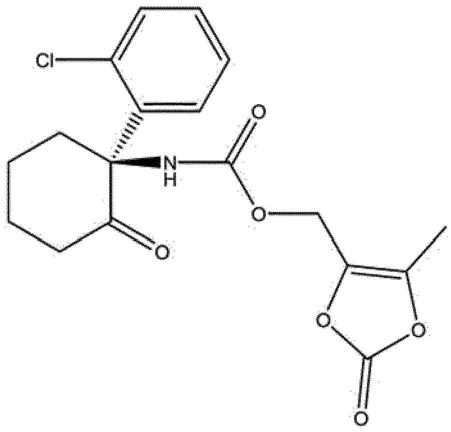

(XIII)

[F14]

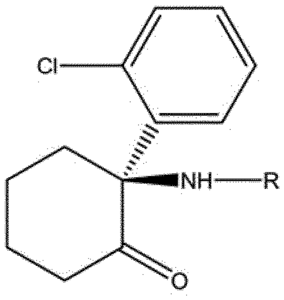

(III)

[F15]

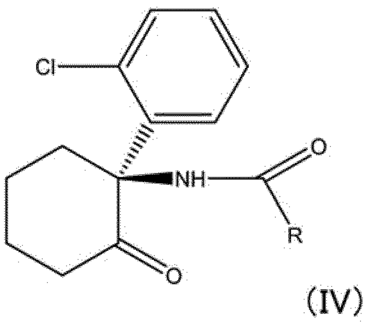

(IV)

[F16]

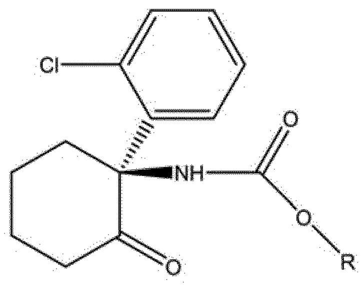

(V)

[F17]

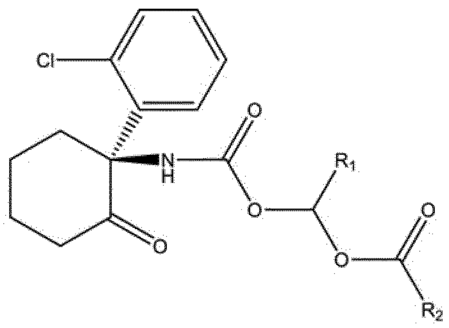

(VI)

[F18]

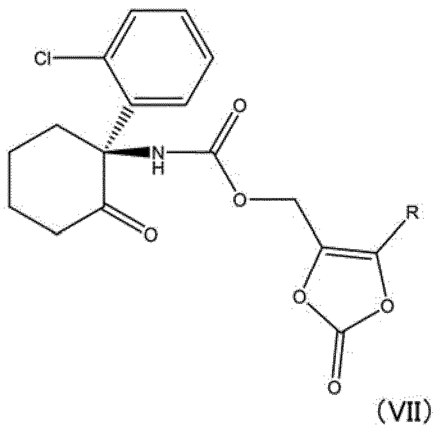

(VII)

[F19]

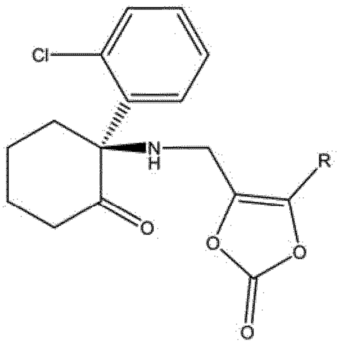

(VIII)

[F20]

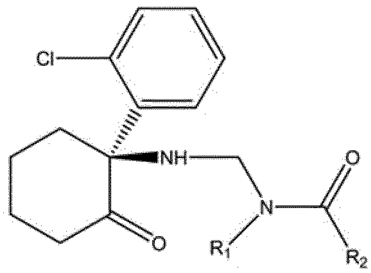

(IX)

[F21]

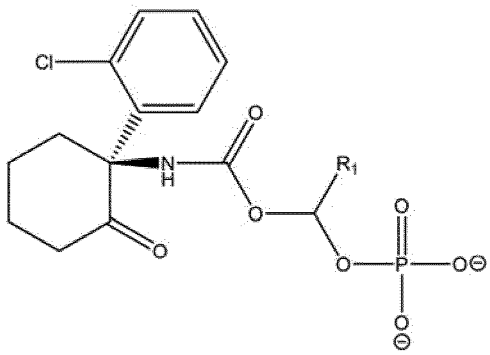

(X)

[F22]

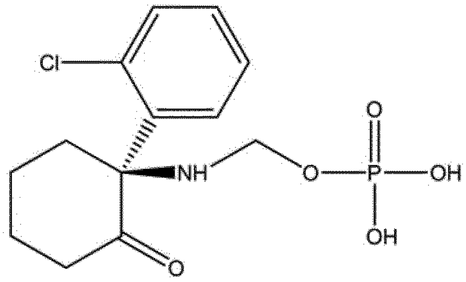

(XI)

[F23]

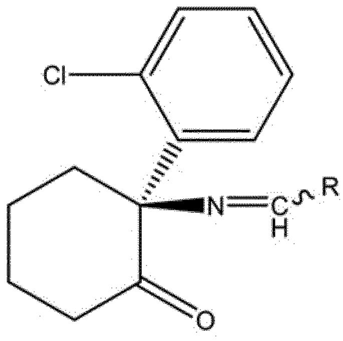

(XII)

[F24]

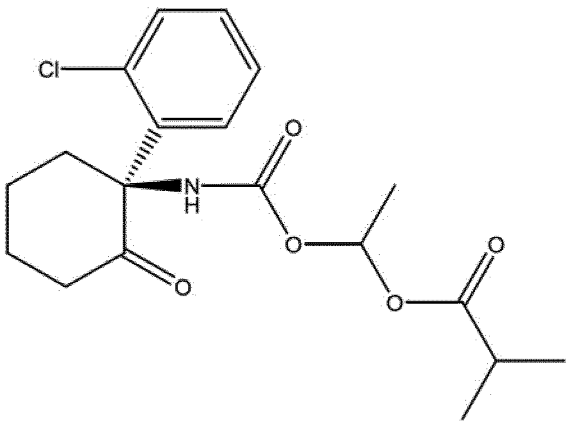

(XIV)

(wherein R represents an alkyl group, an alkoxy group, an aryl group, or an aralkyl group, and $R_1$ and $R_2$ each independently represent an alkyl group, an alkoxy group, an aryl group, or an aralkyl group).

**17.** (5-Methyl-2-oxo-1,3-dioxol-4-yl)methyl-(S)-(1-(2-chlorophenyl)-2-oxocyclohexyl) carbamate.

**18.** 1-((((S)-1-(2-chlorophenyl)-2-oxocyclohexyl)carbamoyl)oxy)ethyl isobutyrate.

**19.** A drug for prevention and/or treatment of a depressive symptom, the drug containing (5-methyl-2-oxo-1,3-dioxol-4-yl)methyl-(S)-(1-(2-chlorophenyl)-2-oxocyclohexyl) carbamate or 1-((((S)-1-(2-chlorophenyl)-2-oxocyclohexyl)carbamoyl)oxy)ethyl isobutyrate.

FIG. 1A

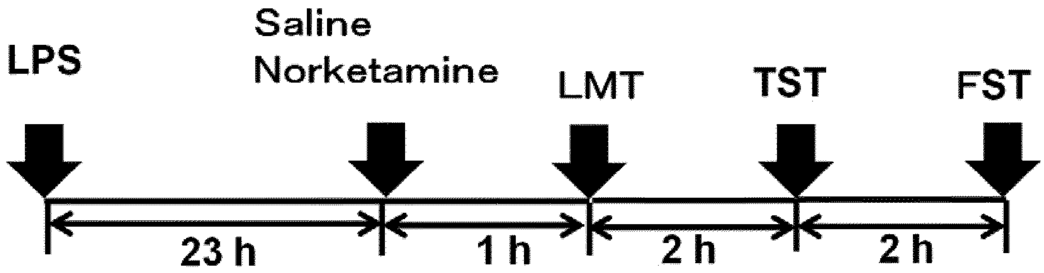
LPS
Saline
Norketamine
LMT
TST
FST
23 h
1 h
2 h
2 h

FIG. 1B

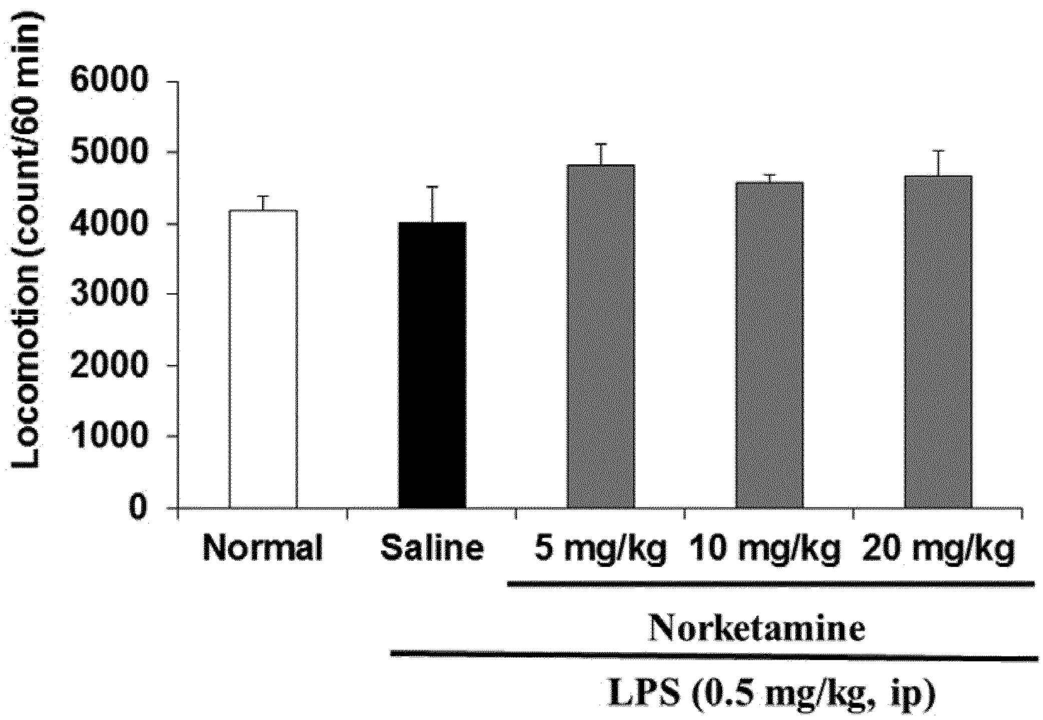
Locomotion (count/60 min)
6000
5000
4000
3000
2000
1000
0
Normal
Saline
5 mg/kg
10 mg/kg
20 mg/kg
Norketamine
LPS (0.5 mg/kg, ip)

FIG. 1C

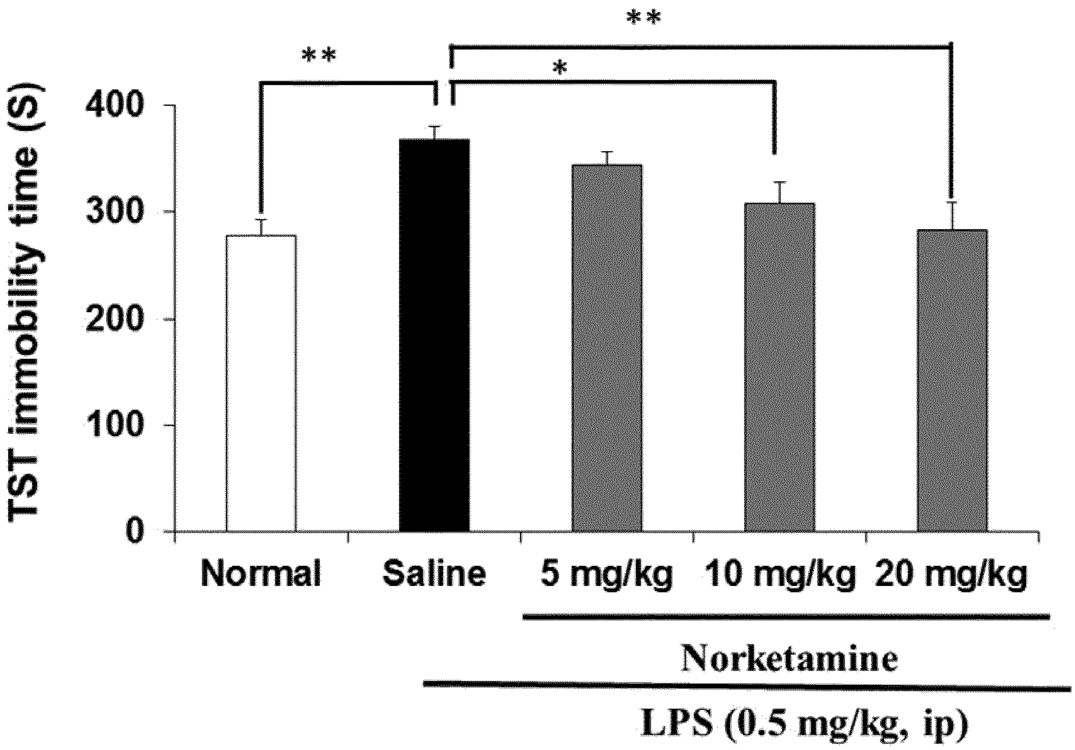
**
*
**
TST immobility time (S)
400
300
200
100
0
Normal
Saline
5 mg/kg
10 mg/kg
20 mg/kg
Norketamine
LPS (0.5 mg/kg, ip)

FIG. 1D

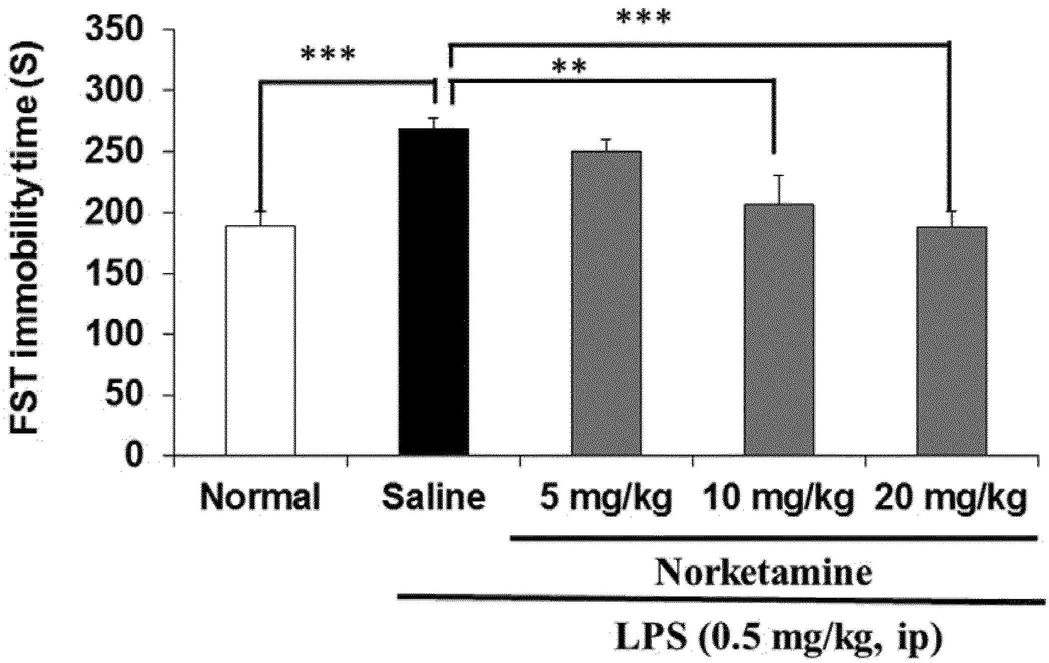
FST immobility time (S)
350
300
250
200
150
100
50
0
***
***
**
Normal
Saline
5 mg/kg
10 mg/kg
20 mg/kg
Norketamine
LPS (0.5 mg/kg, ip)

FIG. 2A

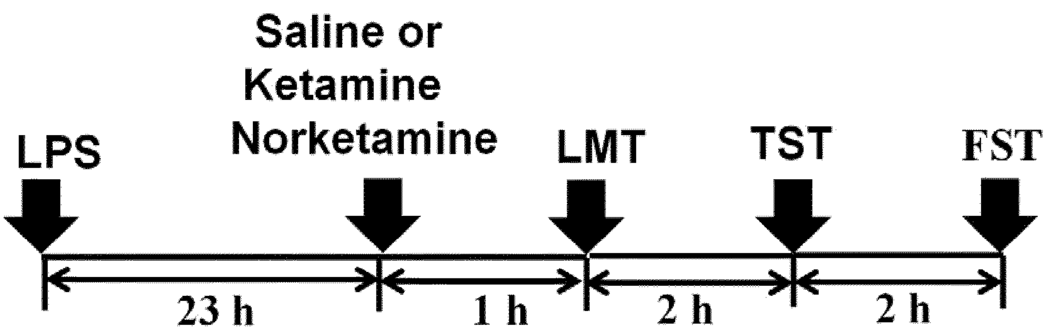
LPS
Saline or
Ketamine
Norketamine
LMT
TST
FST
23 h
1 h
2 h
2 h

FIG. 2B

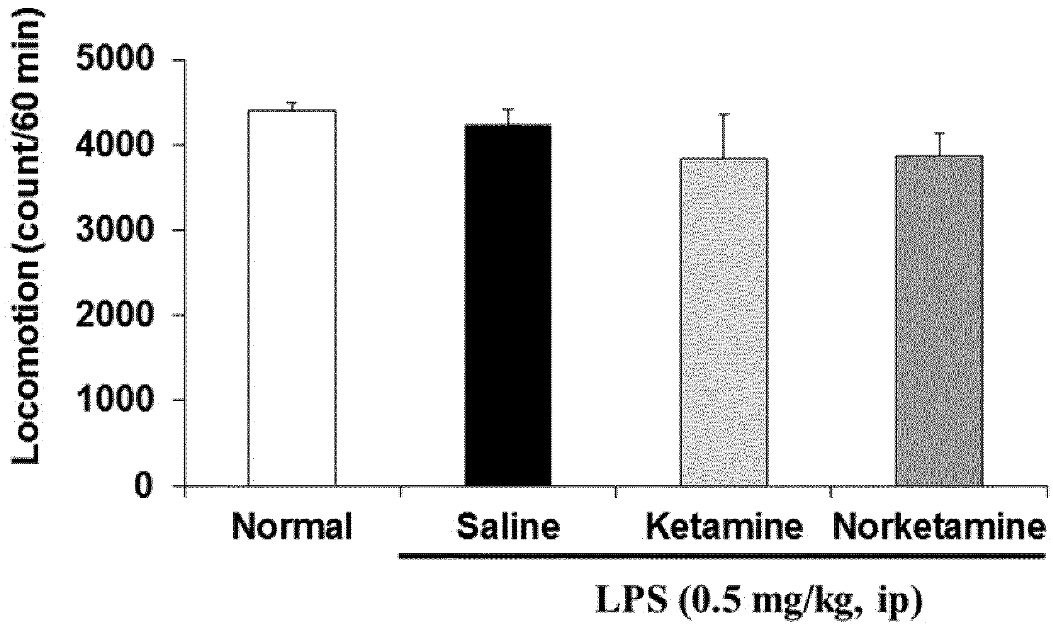
Locomotion (count/60 min)
5000
4000
3000
2000
1000
0
Normal
Saline
Ketamine
Norketamine
LPS (0.5 mg/kg, ip)

FIG. 2C

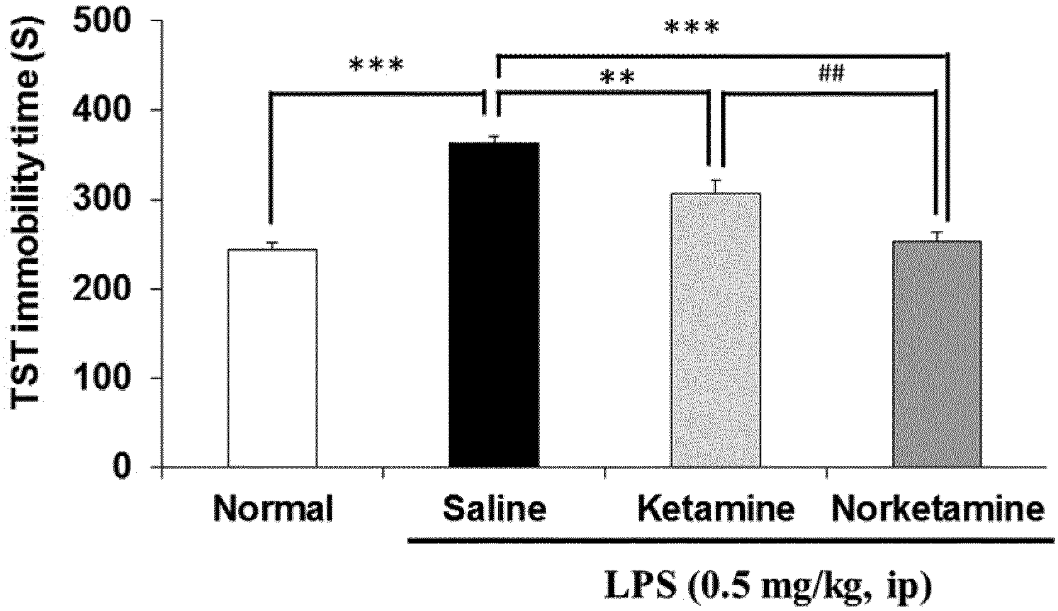
TST immobility time (S)
500
400
300
200
100
0
***
***
**
##
Normal
Saline
Ketamine
Norketamine
LPS (0.5 mg/kg, ip)

FIG. 2D

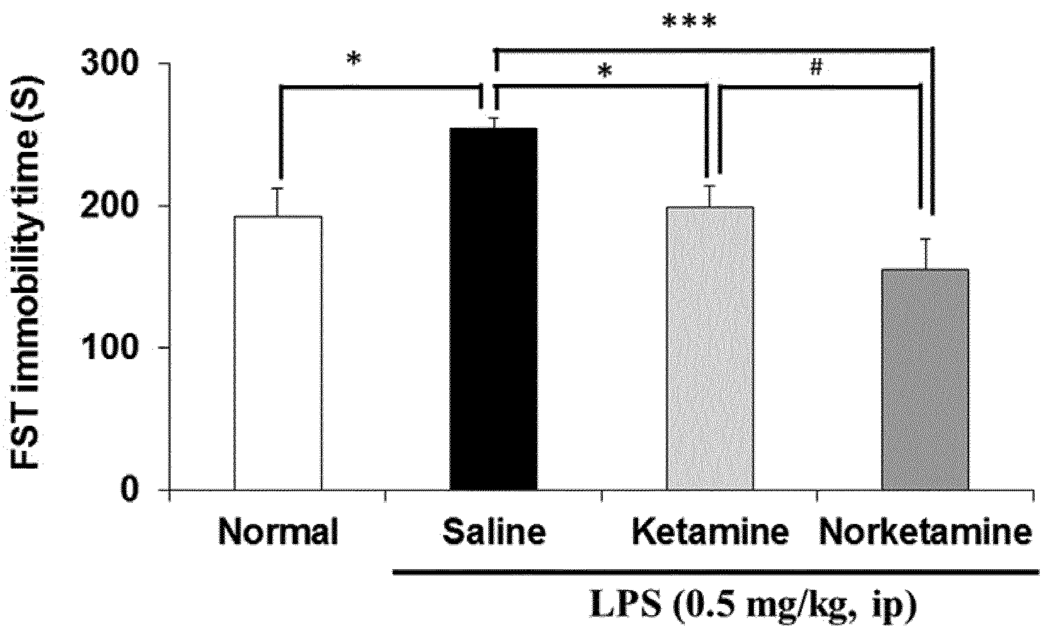

FST immobility time (S)
300
200
100
0
*
*
#
***
Normal
Saline
Ketamine
Norketamine
LPS (0.5 mg/kg, ip)

FIG. 3A

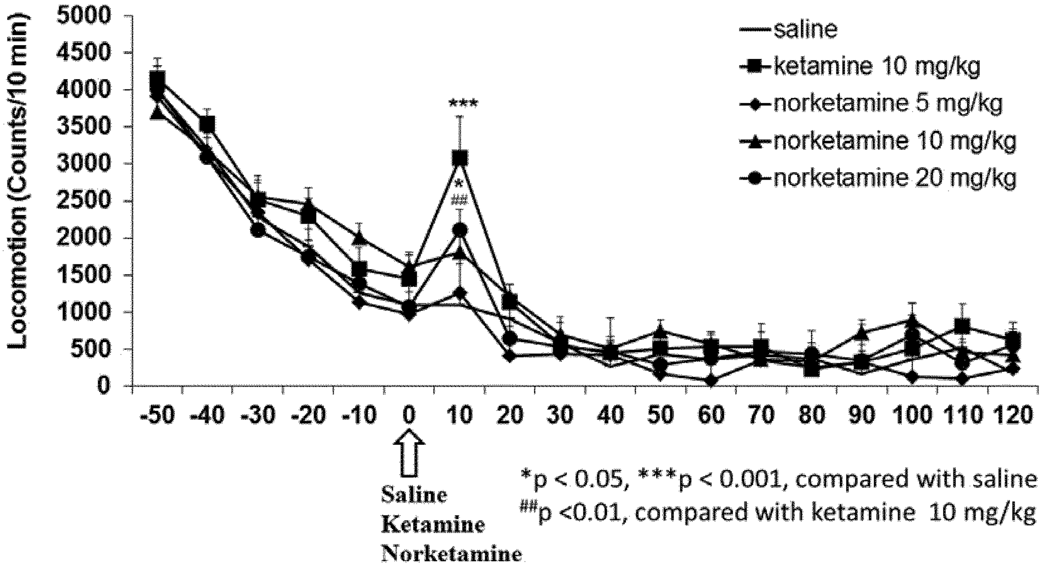
Locomotion (Counts/10 min)
5000
4500
4000
3500
3000
2500
2000
1500
1000
500
0
-50 -40 -30 -20 -10 0 10 20 30 40 50 60 70 80 90 100 110 120
saline
ketamine 10 mg/kg
norketamine 5 mg/kg
norketamine 10 mg/kg
norketamine 20 mg/kg
***
*
##
Saline
Ketamine
Norketamine
*p < 0.05, ***p < 0.001, compared with saline
##p <0.01, compared with ketamine 10 mg/kg

FIG. 3B

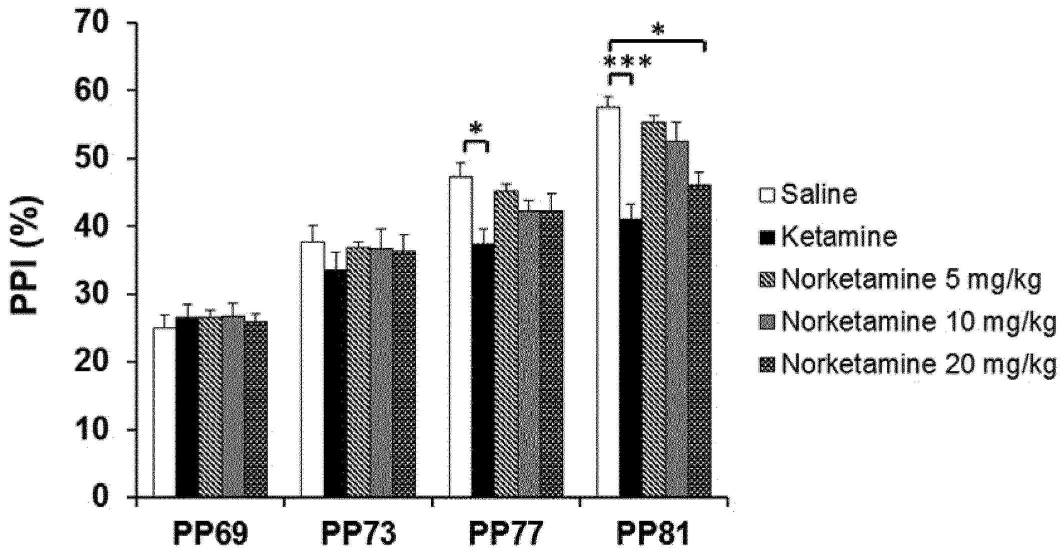
PPI (%)
70
60
50
40
30
20
10
0
PP69
PP73
PP77
PP81
*
***
*
Saline
Ketamine
Norketamine 5 mg/kg
Norketamine 10 mg/kg
Norketamine 20 mg/kg

FIG. 4A

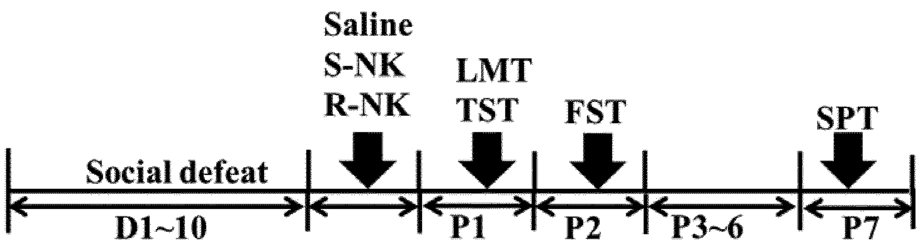


S-NK: S-norketamine. R-NK: R-norketamine. SPT: 1% sucrose preference test.
LMT: locomotion test. TST: tail suspension test. FST: force swimming test.

FIG. 4B

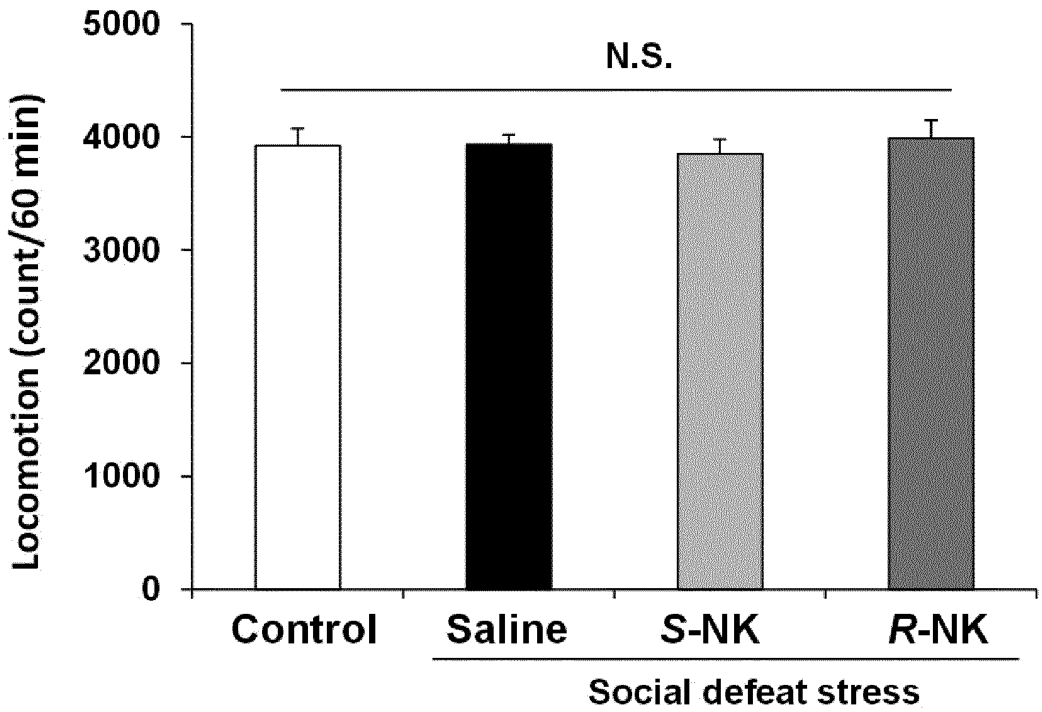

FIG. 4C

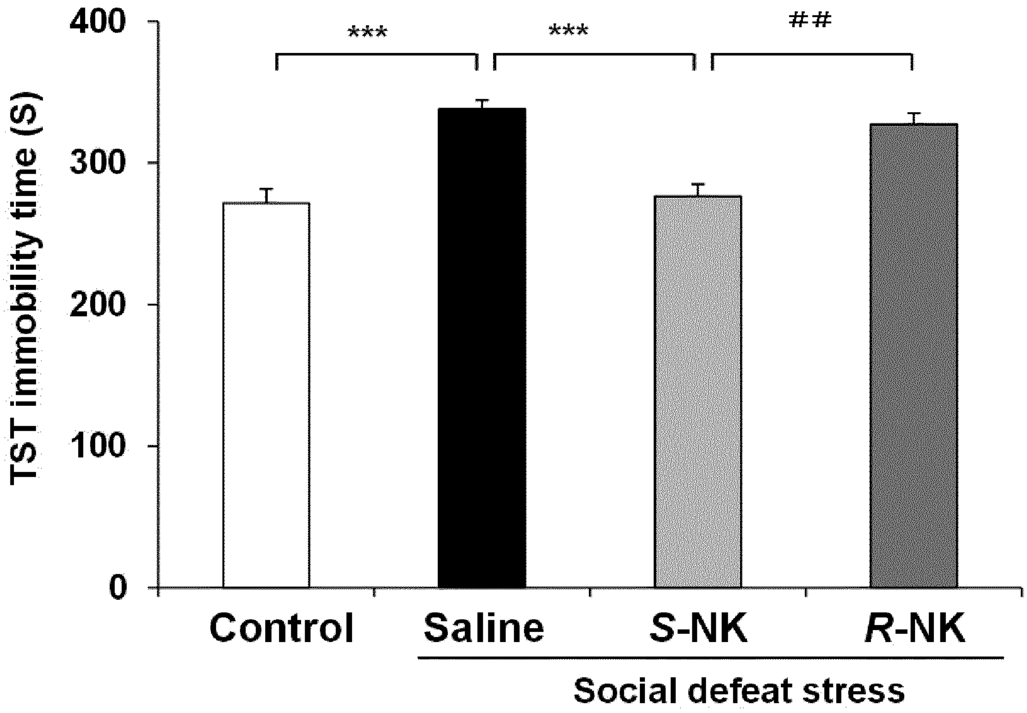

TST immobility time (S)
400
300
200
100
0
***
***
##
Control
Saline
*S*-NK
*R*-NK
Social defeat stress

FIG. 4D

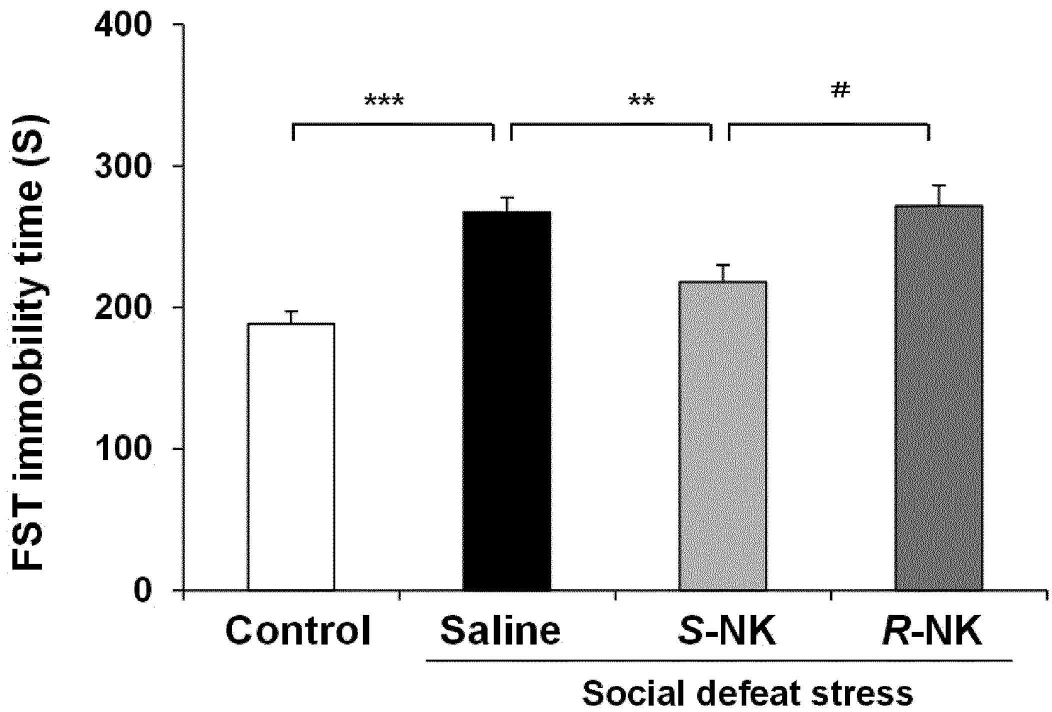

400
300
200
100
0
FST immobility time (S)
***
**
#
Control
Saline
S-NK
R-NK
Social defeat stress

FIG. 4E

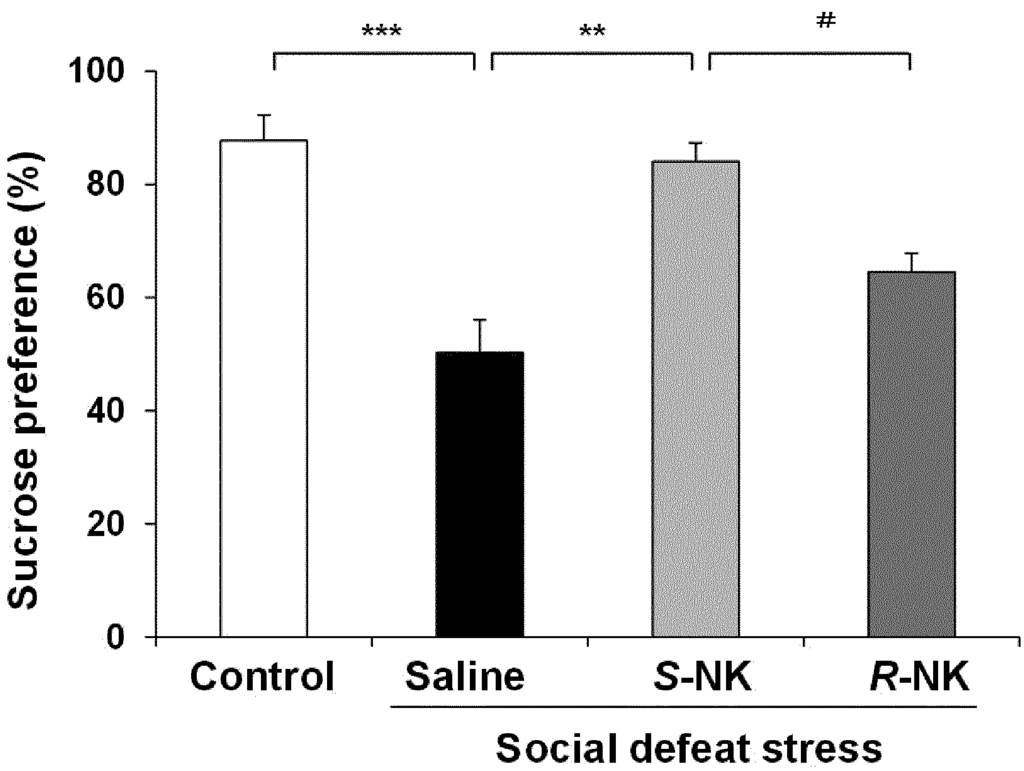
***
**
#
100
80
60
40
20
0
Sucrose preference (%)
Control
Saline
S-NK
R-NK
Social defeat stress

FIG. 4F

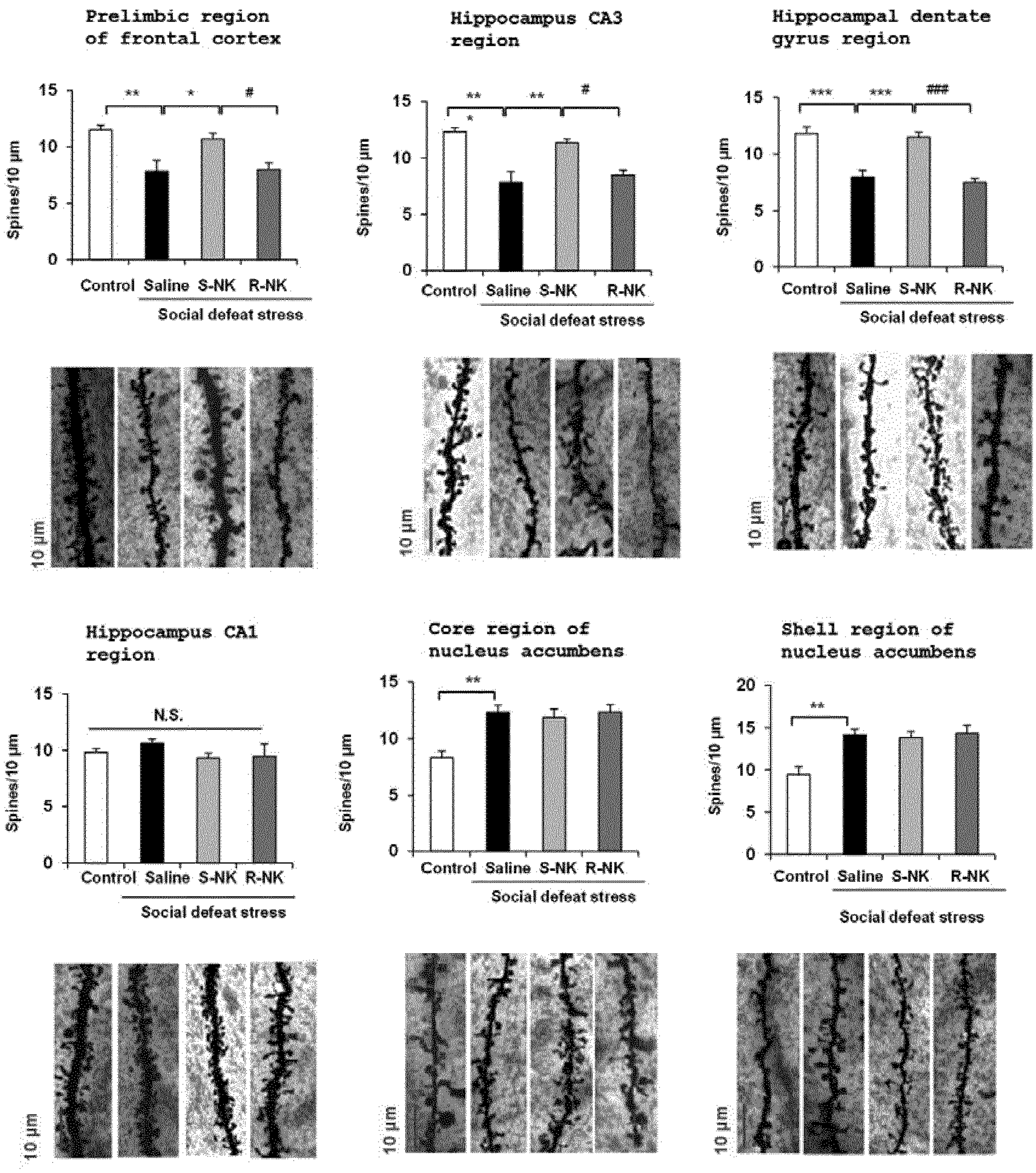
Prelimbic region of frontal cortex
Spines/10 µm
Control
Saline
S-NK
R-NK
Social defeat stress
10 µm
Hippocampus CA3 region
Hippocampal dentate gyrus region
Hippocampus CA1 region
N.S.
Core region of nucleus accumbens
Shell region of nucleus accumbens

FIG. 4G

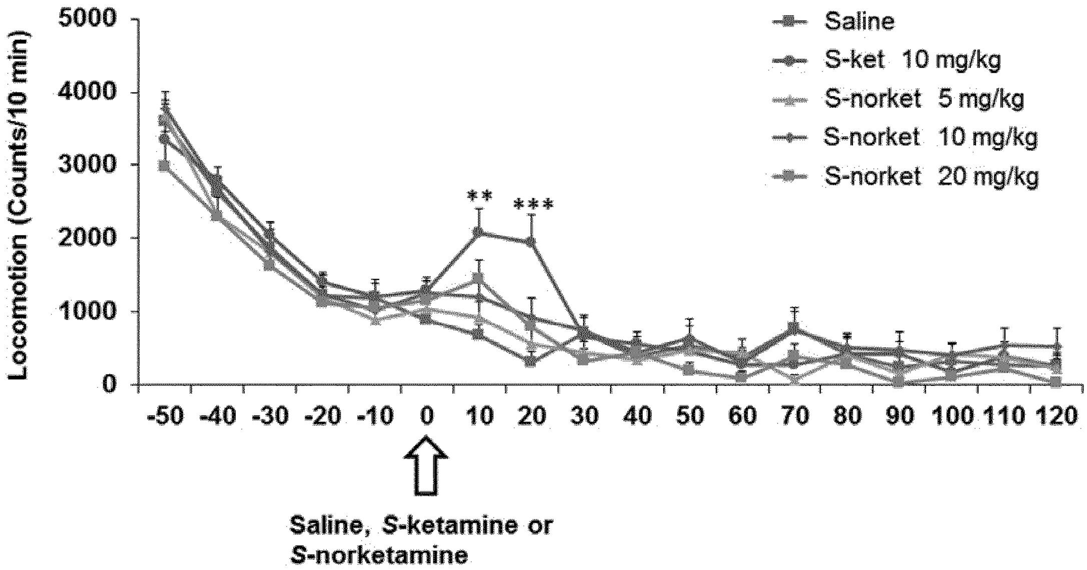
Locomotion (Counts/10 min)
5000
4000
3000
2000
1000
0
-50 -40 -30 -20 -10 0 10 20 30 40 50 60 70 80 90 100 110 120
**
***
Saline
S-ket 10 mg/kg
S-norket 5 mg/kg
S-norket 10 mg/kg
S-norket 20 mg/kg
Saline, S-ketamine or
S-norketamine

FIG. 4H

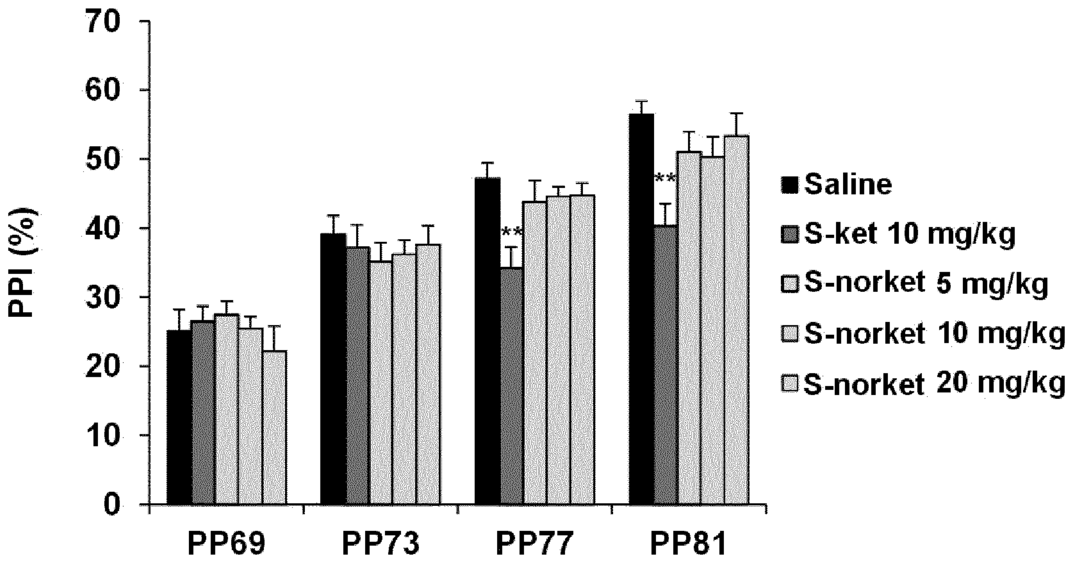
PPI (%)
70
60
50
40
30
20
10
0
PP69
PP73
PP77
PP81
**
**
Saline
S-ket 10 mg/kg
S-norket 5 mg/kg
S-norket 10 mg/kg
S-norket 20 mg/kg

FIG. 4I

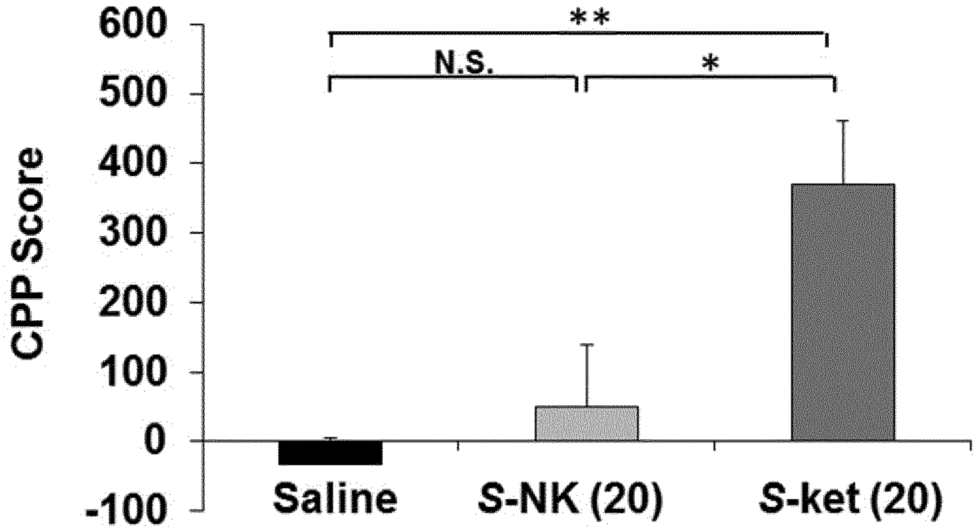

**
N.S.
*
600
500
400
300
200
100
0
-100
CPP Score
Saline
S-NK (20)
S-ket (20)


FIG. 5

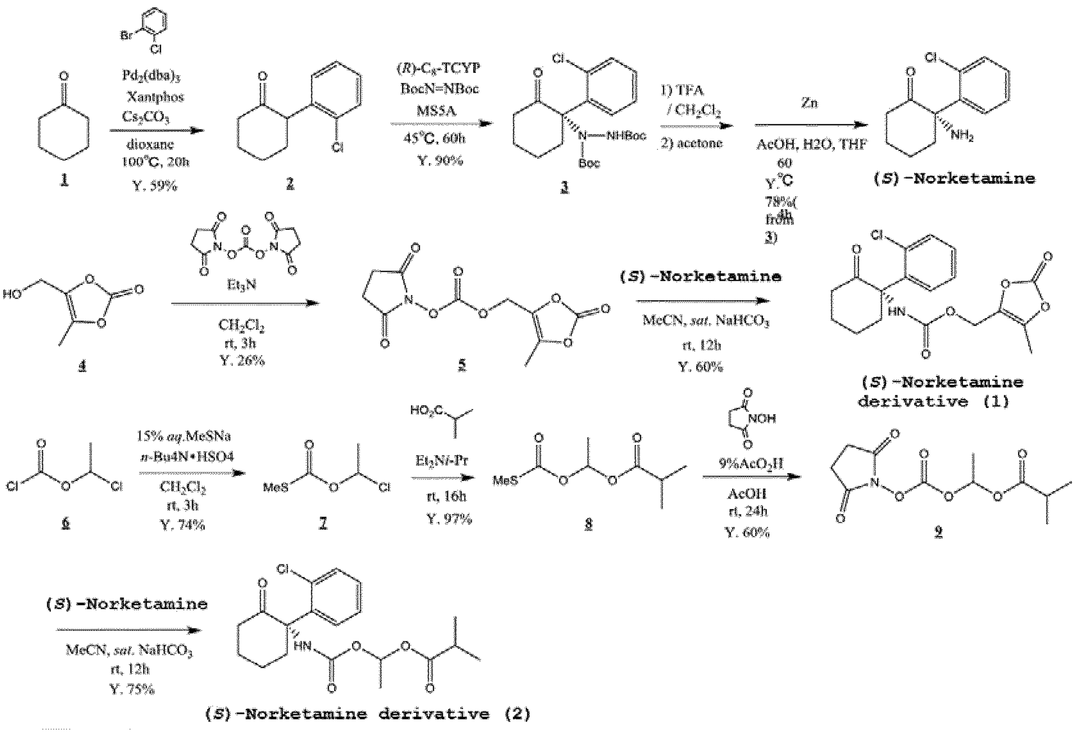

$Pd_2(dba)_3$
Xantphos
$Cs_2CO_3$
dioxane
100°C, 20h
Y. 59%
1
2
(R)-$C_8$-TCYP
BocN=NBoc
MS5A
45°C, 60h
Y. 90%
3
1) TFA
/ $CH_2Cl_2$
2) acetone
Zn
AcOH, H2O, THF
60
Y.°C
78%(
from
3)
4h
(S)-Norketamine
$Et_3N$
$CH_2Cl_2$
rt, 3h
Y. 26%
4
5
(S)-Norketamine
MeCN, sat. $NaHCO_3$
rt, 12h
Y. 60%
(S)-Norketamine
derivative (1)
15% aq.MeSNa
n-Bu4N•HSO4
$CH_2Cl_2$
rt, 3h
Y. 74%
6
7
$Et_3N$i-Pr
rt, 16h
Y. 97%
8
9%$AcO_2H$
AcOH
rt, 24h
Y. 60%
9
(S)-Norketamine
MeCN, sat. $NaHCO_3$
rt, 12h
Y. 75%
(S)-Norketamine derivative (2)

FIG. 6A

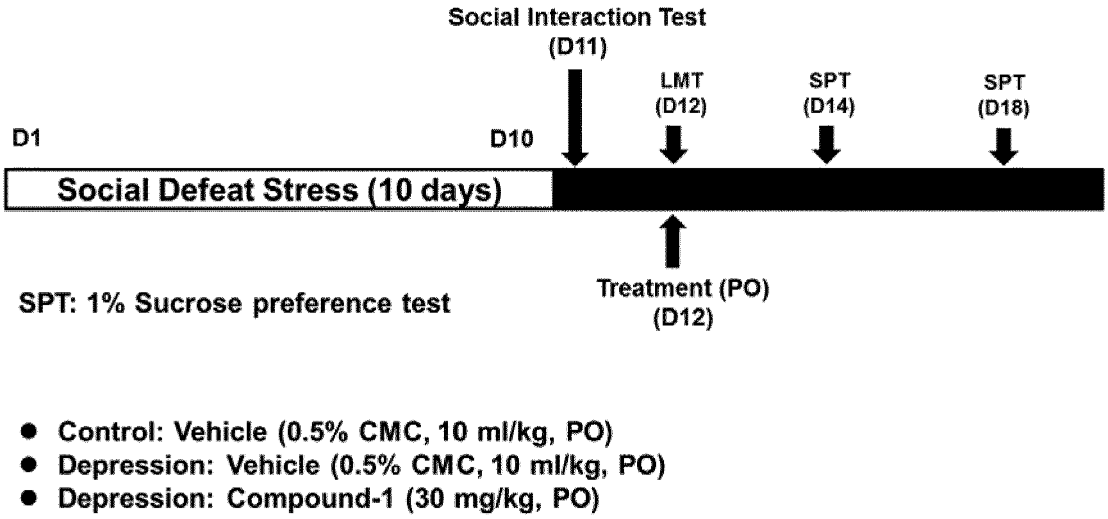
Social Interaction Test
(D11)
LMT
(D12)
SPT
(D14)
SPT
(D18)
D1
D10
Social Defeat Stress (10 days)
Treatment (PO)
(D12)
SPT: 1% Sucrose preference test
Control: Vehicle (0.5% CMC, 10 ml/kg, PO)
Depression: Vehicle (0.5% CMC, 10 ml/kg, PO)
Depression: Compound-1 (30 mg/kg, PO)

FIG. 6B

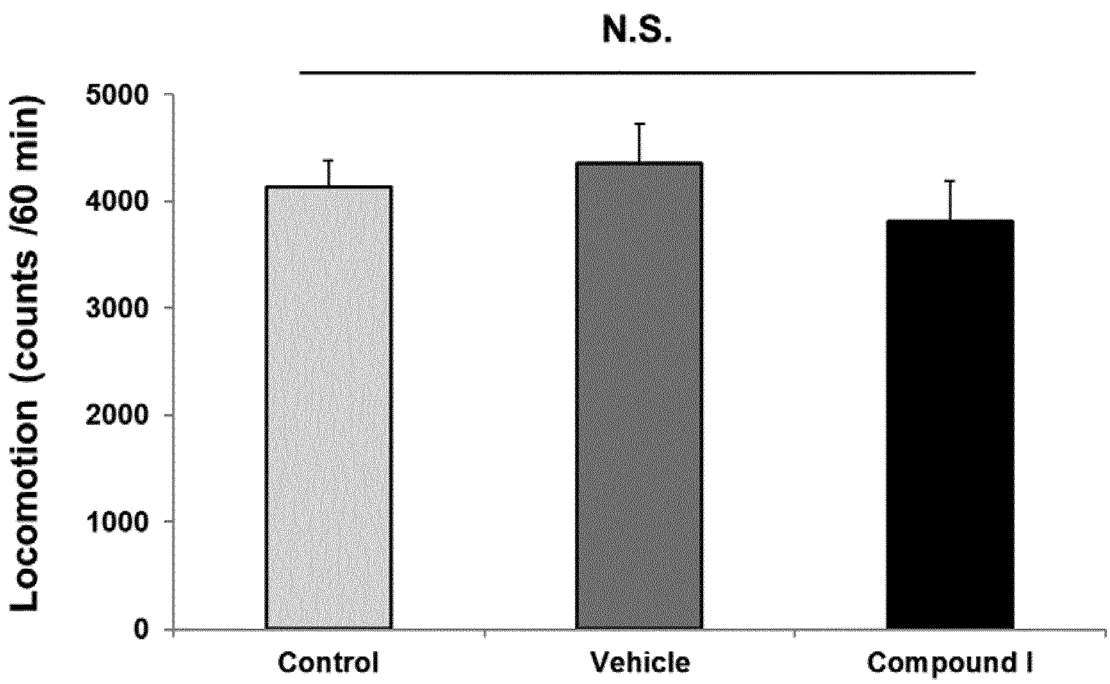
N.S.
Locomotion (counts /60 min)
5000
4000
3000
2000
1000
0
Control
Vehicle
Compound I

FIG. 6C

Three days after administration

Seven days after administration

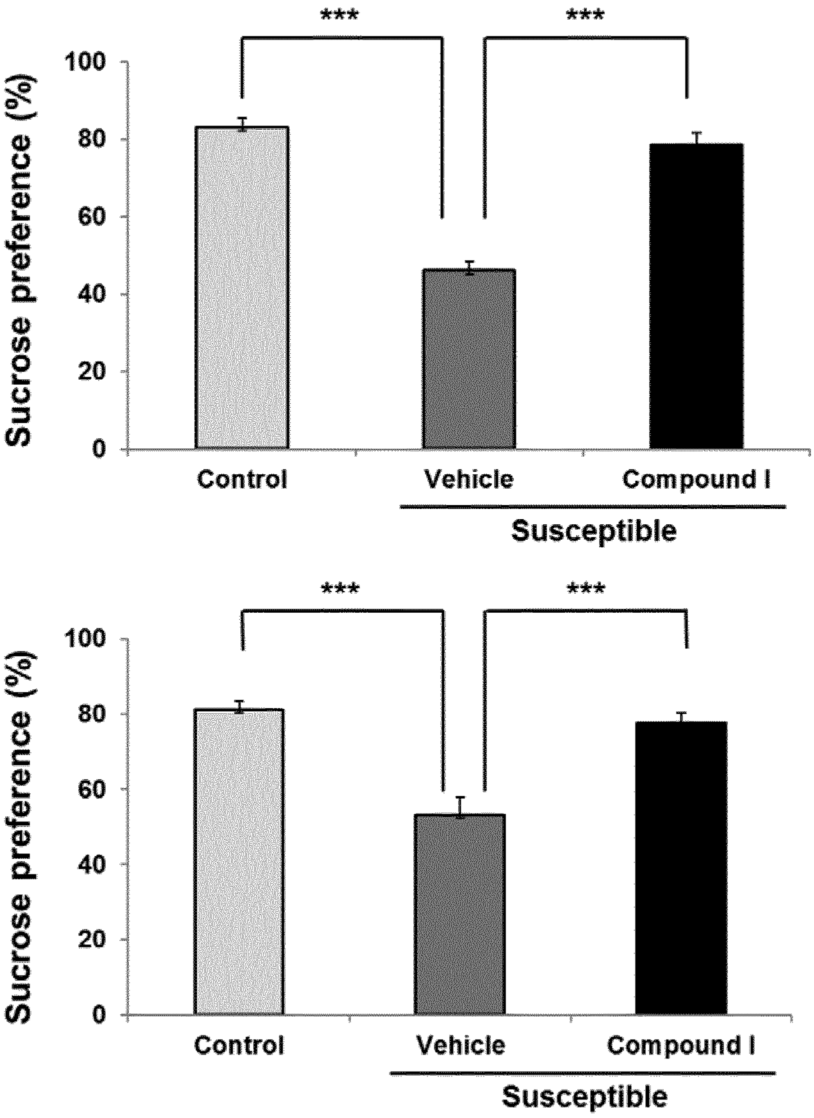

**INTERNATIONAL SEARCH REPORT**

International application No. PCT/JP2017/038826

A. CLASSIFICATION OF SUBJECT MATTER
Int. Cl. A61K31/135(2006.01)i, A61K31/357(2006.01)i, A61K31/661(2006.01)i, A61P25/22(2006.01)i, A61P25/24(2006.01)i, C07C211/29(2006.01)i, C07D317/24(2006.01)i

B. FIELDS SEARCHED
Minimum documentation searched (classification system followed by classification symbols)
Int. Cl. A61K31/135, A61K31/357, A61K31/661, A61P25/22, A61P25/24, C07C211/29, C07D317/24

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br><br>Y<br><br>A | JASKARAN, B. S, et al., Intravenous Esketamine in Adult Treatment-Resistant Depression: A Double-Blind, Double-Randomization, Placebo-Controlled Study, Biological Psychiatry, 15 September 2016, Vol. 80, No. 6, ISSN 0006-3223, pp. 424-431, abstract, fig. 1, p. 424, left column, paragraph 2 | 1-3, 5-7, 9-11, 13-16<br>1-3, 5-7, 9-11, 13-16<br>4, 8, 12, 17-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| | |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2017/038826

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | GISLAINE, Z. R. et al., A single dose of S-ketamine induces long-term antidepressant effects and decreases oxidative stress in adulthood rats following maternal deprivation, Developmental Neurobiology, 2015 Nov, Vol. 75, No. 11, pp. 1268-1281, Abstract, fig. 2 | 1-3, 5-7, 9-11, 13-16<br><br>4, 8, 12, 17-19 |
| X<br><br>Y<br><br>A | WO 2016/044150 A1 (JANSSEN PHARMACEUTICA NV) 24 March 2016, claims, paragraphs, embodiments, table 2, p. 6, line 3-6 & JP 2017-528483 A claims, paragraphs, embodiments, table 2, [0015] & US 2016/0074340 A1 & EP 3193853 A1 & KR 10-2017-0054470 A & CN 107208133 A | 1-3, 5-7, 9-11, 13-16<br>1-3, 5-7, 9-11, 13-16<br>4, 8, 12, 17-19 |
| Y<br><br>A | RAJIB, K. P. et al., [R, S)-Ketamine Metabolites (R,S)-norketamine and (2S,6S)-hydroxynorketamine Increase the Mammalian Target of Rapamycin Function, Anesthesiology, 2014 Jul, Vol. 121, No. 1, ISSN 0003-3022, p. 149-159, abstract, p. 152, right column, line 52 to p. 153, right column, line 2, p. 153, right column, last line to page 155, left column, line 4, fig. 6 (B), (E) | 1-3, 5-7, 9-11, 13-16<br><br>4, 8, 12, 17-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016210749 A
- JP 2015078181 A
- WO 2015037248 A
- US 6040479 A


### Non-patent literature cited in the description


- **HASHIMOTO K.** Emerging role of glutamate in the pathophysiology of major depressive disorder. *Brain Res. Rev.,* 2009, vol. 61, 105-23
- **BERMAN RM ; CAPPIELLO A, AN ; A, OREN DA ; HENINGER GR ; CHARNEY DS ; KRYSTAL JH.** Antidepressant effects of ketamine in depressed patients. *Biol. Psychiatry,* 2000, vol. 47, 351-4
- **ZARATE CA, JR ; SINGH JB ; CARLSON PJ ; BRUTSCHE NE ; AMELI R ; LUCKENBAUGH DA ; CHARNEY DS ; MANJI HK.** A randomized trial of an N-methyl-D-aspartate antagonist in treatment-resistant major depression. *Arch. Gen. Psychiatry,* 2006, vol. 63, 856-64
- **DIAZGRANADOS N ; IBRAHIM L ; BRUTSCHE NE ; NEWBERG A ; KRONSTEIN P ; KHALIFE S ; KAMMERER WA ; QUEZADO Z ; LUCKENBAUGH DA ; SALVADORE G.** A randomized add-on trial of an N-methyl-D-aspartate antagonist in treatment-resistant bipolar depression. *Arch. Gen. Psychiatry,* 2010, vol. 67, 793-802
- **BLOCH MH ; WASYLINK S ; LANDEROS-WEISENBERGER A ; PANZA KE ; BILLINGSLEA E ; LECKMAN JF ; KRYSTAL JH ; BHAGWAGAR Z ; SANACORA G ; PITTENGER C.** Effects of ketamine in treatment-refractory obsessive-compulsive disorder. *Biol. Psychiatry,* 2012, vol. 72 (11), 964-970
- **RODRIGUEZ CI ; KEGELES LS ; LEVINSON A ; FENG T ; MARCUS SM ; VERMES D ; FLOOD P ; SIMPSON HB.** Randomized Controlled Crossover Trial of Ketamine in Obsessive-Compulsive Disorder: Proof-of-Concept. *Neuropsychopharmacology,* 2013, vol. 38 (12), 2475-2483
- **FEDER A ; PARIDES MK ; MURROUGH JW ; PEREZ AM ; MORGAN JE ; SAXENA S ; KIRKWOOD K ; AAN HET ROT M ; LAPIDUS KA ; WAN LB.** Efficacy of intravenous ketamine for treatment of chronic posttraumatic stress disorder: a randomized clinical trial. *JAMA Psychiatry,* 2014, vol. 71, 681-688
- **KRYSTAL JH ; SANACORA G ; DUMAN RS.** Rapid-acting glutamatergic antidepressants: the path to ketamine and beyond. *Biol. Psychiatry,* 2013, vol. 73, 1133-41
- **WINK LK ; O'MELIA AM ; SHAFFER RC ; PEDAPATI E ; FRIEDMANN K ; SCHAEFER T ; ERICKSON CA.** Intranasal ketamine treatment in an adult with autism spectrum disorder. *J. Clin. Psychiatry,* 2014, vol. 75 (8), 835-836
- **ZHANG JC ; LI SX ; HASHIMOTO K.** R(-)-Ketamine shows greater potency and longer lasting antidepressant effects than S(+)-ketamine. *Pharmacol. Biochem. Behav.,* 2014, vol. 116, 137-141
- **YANG C ; SHIRAYAMA Y ; ZHANG JC ; REN Q ; YAO W ; MA M ; DONG C ; HASHIMOTO K.** R-Ketamine: a rapid-onset and sustained antidepressant without psychotomimetic side effects. *Transl. Psychiatry,* 2015, vol. 5, e632
- **EBERT B ; MIKKELSEN S ; THORKILDSEN C ; BORDBJERG FM.** Norketamine, the main metabolite of ketamine, is a non-competitive NMDA receptor antagonist in the rat cortex and spinal cord. *Eur. J. Pharmacology,* 1997, vol. 333, 99-104
- **SARAT K ; SIWEK A ; STAROXICZ G ; LIBROWSKI T ; NOWAK G ; DRABIK U ; GAJDOSZ R ; POPIK P.** Antidepressant-like effects of ketamine, norketamine and dehydronorketamine in forced swim test: Role of activity at NMDA receptor. *Neuropharmacology,* 2015, vol. 99, 301-307
- **MA M ; REN Q ; ZHANG JC ; HASHIMOTO K.** Effects of brilliant blue G on serum levels of tumor necrosis factor-alpha and depression-like behaviors in mice after administration of lipopolysaccharide. *Clin. Psychopharmacol. Neurosci.,* 2014, vol. 12, 31-36
- **ZHANG JC ; WU J ; FUJITA Y ; YAO W ; REN Q ; YANG C ; LI SX ; SHIRAYAMA Y ; HASHIMOTO K.** Antidepressant effects of TrkB ligands on depression-like behavior and dendritic changes in the hippocampus and nucleus accumbens after inflammation. *Int. J. Neuropsychopharmacol.,* 2015, vol. 18

- **YAO W ; ZHANG JC ; DONG C ; ZHUANG C ; HIROTA S ; INANAGA K ; HASHIMOTO K.** Effects of amycenone on serum levels of tumor necrosis factor-alpha and depression-like behaviors in mice after administration of lipopolysaccharide. *Pharmacol. Biochem. Behav.,* 2015, vol. 136, 7-12
- **BIERMANN M ; ZHENG G ; HOJAHMAT M ; MOSKALEV NV ; CROOKS PA.** Asymmetric synthesis of (S)- and (R)-norketamine via Sharpless asymmetric dihydroxylation/Ritter amination sequence. *Tetrahedron Letters,* 2015, vol. 56, 2608-2610
- **RAUTIO J ; KUMPULAINEN H ; HEIMBACH T ; OLIYAI R ; OH D ; JARVINEN T ; SAVOLAINEN J.** Prodrugs: design and clinical applications. *Nat. Rev. Drug Discov.,* 2008, vol. 7 (3), 255-270
- **SIMPLICIO AL ; CLANCY JM ; GILMER.** Prodrugs for amines. *Molecules,* 2008, vol. 13, 519-547
- Remington's Pharmaceutical Sciences. Mack Publishing Company, 1970
- *Nature,* 2016, vol. 533, 481